# EUROPEAN PATENT SPECIFICATION

(11) **EP 4 181 864 B1**
(45) Date of publication and mention of the grant of the patent: **19.08.2026**
(21) Application number: 21752602.9
(22) Date of filing: 14.07.2021
(51) Int. Cl.: A61K 8/06, A61K 8/73, A61Q 5/02, A61Q 5/10, A61Q 5/12

(54) **COSMETIC FORMULATIONS FOR HAIR TREATMENT WITH IMPROVED PROPERTIES**
KOSMETISCHE FORMULIERUNGEN ZUR HAARBEHANDLUNG MIT VERBESSERTEN EIGENSCHAFTEN
FORMULATIONS COSMÉTIQUES POUR LE TRAITEMENT CAPILLAIRE AYANT DES PROPRIÉTÉS AMÉLIORÉES

(30) Priority: 17.07.2020 IT 202000017485
(43) Date of publication of application: 24.05.2023
(73) Proprietor: Davines S.p.A., 43126 Parma (IT)
(72) Inventor: GOI, Paolo, 20064 GORGONZOLA (IT); NICOLI, Margherita, 43126 PARMA (IT); CACCIA, Teresa, 43123 PARMA (IT); GALLO, Michela, 43010 FONTEVIVO (IT); DE CARNE, Beatrice, 43126 PARMA (IT)
(74) Representative: Metroconsult Srl
(86) International application number: PCT/IT2021/050218
(87) International publication number: WO 2022/013900

(56) References cited:
- EP-B1- 3 081 208
- EP-B1- 3 283 052
- WO-A1-2008/057985
- WO-A1-2013/154675
- WO-A1-2020/025404
- CN-B- 101 862 281
- KARPPINEN ANNI: "How to use MFC in cosmetic products? Interview with Cosmacon & Rainer Kröpke", 11 September 2016 (2016-09-11), XP055793399, Retrieved from the Internet <URL:https://www.exilva.com/blog/how-to-use-mfc-in-cosmetic-products-interview-with-cosmacon-and-rainer-kröpke> [retrieved on 20210408]

## Description

On account of an increased awareness of the damage caused to the environment by non-biodegradable synthetic derivatives for cosmetic use, there has arisen an increasing demand for raw materials of natural origin, which are sustainable and eco-friendly, but at the same time offer high levels of performance, for the development of cosmetic formulations to be applied on hair. It is known that many cosmetic compositions for hair care are oil-in-water (O/W) emulsions that require the presence of additives capable of improving the stability of the emulsions themselves. Generally, such functions are ensured by the presence of synthetic polymers of various nature, in particular (co)polyacrylates of various types, namely, homopolymers, copolymers, or crosslinked, which are well known and widely used in the cosmetics sector, such as the ones known by the names of Pemulen, Carbomer, and Carbosperse. One example of this is Pemulen^{®} TR1, which belongs to the category of the acrylic polymers known as emulsifiers-gellifiers: these are high-molecular-weight crosslinked polymers of acrylic acid that have a small lipophilic portion and a large hydrophilic portion. An example of Carbomer is Carbopol^{®} Ultrez-21 or Ultrez-20: these are acrylate/C10-C30 alkyl acrylate crosspolymers with viscosifying and gelling properties; as compared to other acrylates, they present self-hydration properties. Finally, we recall Carbosperse^{®} K775 and 776: these are hydrosoluble acrylate copolymers.

Other compositions exist for hair care that usually envisage the presence of other synthetic compounds. Formulations with conditioning action belong in this category. Typical cosmetic ingredients with conditioning effect are cationic surfactants in combination with long-chain fatty alcohols, and/or with other lipid components. The main function of conditioners, which have a positive charge, consists in neutralising the anionic charge of the surface of the hair. Cetrimonium chloride and behentrimonium methosulphate are typical examples of cationic surfactants used in numerous formulations for hair. Other ingredients are also used, of a synthetic nature or of a partially synthetic nature, such as dimethyl stearamine, stearyl dimethyl amine and, very frequently, cationic polymers, such as Polyquaternium-10 or Polyquaternium-37.

All the chemical compounds mentioned above, i.e., those with stabilising effect and those with conditioning effect, present high performance but have a marked environmental impact; they are in fact characterised by low levels or absence of biodegradability. In addition, conditioning chemical compounds are also characterised by acute toxicity for aquatic organisms (category II and III of the CLP classification) and in the case where these degrees of toxicity are associated with low-biodegradability data, use of the conditioning raw materials can lead to effects of acute toxicity of long duration; for this reason, in view of the continuous demand for hair-care products that present increasingly high performance and are sustainable, there is a constant search for cosmetic ingredients that inherently feature characteristics of performance and sustainability.

On the other hand, a material of natural origin is known in the literature, such as microfibrillated cellulose (MFC), the properties of which will be described hereinafter. We have now found that microfibrillated cellulose (MFC) can replace at least in part, if not totally, the (co)polyacrylates referred to above, in this way enabling preparation of eco-sustainable formulations for hair care. It has moreover been surprisingly found that MFC can replace, in part or totally, both (co)polyacrylates used as stabilisers and polymeric quaternary-ammonium salts used as conditioners in so far as MFC simultaneously bestows in an effective way to cosmetic formulations for hair care both stabilising properties (typical of co-polyacrylates) and conditioning properties (typical of the polymeric quaternary-ammonium salts described above).

This will be clearly highlighted in the experimental part, where it will be shown that formulations for hair care containing MFC moreover present an increased strengthening effect on hair and a long-lasting styling.

Microfibrillated cellulose (MFC) is already used in the cosmetic field, mainly as gelling or suspending agents: WO2013031030 describes MFC as a gelling agent in sheet masks; WO2013094077 describes the use of MFC in the production of the material of sheet masks; EP2307100 describes the use of MFC as rheology modifier in liquid detergent formulations; and finally, EP3283052 describes cosmetic compositions containing MFC useful for skin care, in particular as anti-wrinkle products.

Document "How to use MFC in cosmetic products? Interview with Cosmacon & Rainer Kropke" (www.exilva.com/blog/how-to-use-mfc-in-cosmetic-products-interview-with-cosmacon-and-rainer-kr%C3%B6pke) discloses uses of microfibrillated cellulose in cosmetic products, such as oil in water emulsions, water in oil emulsions, gels, cream products (aqueous lip balms), shampoos, conditioners, peeling products.

In view of the above, the invention refers to a use of microfibrillated cellulose (MFC) as a conditioning and strengthening agent of hair, according to independent claim 1. Advantageous aspects of the invention are disclosed in the dependent claims.

In a first embodiment of the present invention, the cosmetic composition for hair care is a shampoo with the further benefit of improved stabilisation of the foam.

As regards the shampoo, the stability of a shampoo containing a mixture of anionic and non-ionic surfactants with stabilising natural polymers (xanthan gum, sclerotium gum) was tested. In particular, a product containing 0.5 wt% of microfibrillated cellulose was tested and compared with similar formulations without microfibrillated cellulose and with similar formulations containing 0.5 wt% of polyacrylates (acrylates/C10-30 alkyl acrylate crosspolymer, Carbomer).

From the tests conducted it has been found that the formulation containing MFC is significantly better than the formulations containing the synthetic polymers referred to above, both from the standpoint of stability and from the standpoint of combability; there has also been found, for MFC, when in association with a surfactant, the property of foam-stabilising booster.

In a second embodiment of the present invention, the cosmetic composition of the present invention is a leave-on hair-conditioning balm for hair care. The stability of a leave-on hair-conditioning balm containing 0.5 wt% of microfibrillated cellulose was tested, the comparison was carried out with similar formulations without microfibrillated cellulose and with similar formulations containing 0.5 wt% of a cationic synthetic conditioner, namely, a polymeric quaternary-ammonium salt derived from ethyl methacrylate, i.e., poly-(2-(methacryloxy)-ethyl)-trimethylammonium chloride (Polyquaternium-37), and the polymeric quaternary-ammonium salt of hydroxyethyl cellulose (HEC) reacted with trimethyl ammonium substituted epoxide (Polyquaternium-10).

Both of the formulations containing MFC prove superior in terms of stability, and, as regards application on hair present, improved properties of combability, long-lasting styling, and strength as compared to the similar products containing synthetic polymers.

According to a third embodiment of the present invention, the cosmetic composition for hair care is a composition for dyeing hair, where the inclusion of MFC in the formula entails, in addition to the effects of increase in stability of the formulation and improvement of combability, the further benefit of an improved anti-slip property, albeit in the presence of pigments, and a strengthening effect. The properties of the composition for dyeing hair made up of two formulations were tested: a dyeing formulation that, at the moment of use, is mixed with a second oxidising formulation. The dyeing formulation tested was made up of 0.5 wt% of MFC dispersed in water; the dyeing formulation tested contained various additives, amongst which emulsifiers, pH correctors, buffers, antioxidants, chelating agents, and pigments. The oxidant formulation tested was made up of 0.75 wt% of MFC dispersed in water; the oxidant formulation tested contained various additives, amongst which an oxidant.

As regards the stability tests, the dyeing and oxidant formulations were tested separately; as regards, instead, the tests combability, ultimate strength, and anti-slip properties, it was decided to test the overall formulation, obtained by mixing in equal parts the dyeing formulation and the oxidant formulation, in order to better simulate the actual behaviour of these products. The formulations were tested in relation to similar formulations without microfibrillated cellulose and similar formulations, already present on the market not containing MFC, and the tests revealed improved performance in terms of combability, anti-slip properties, strengthening effect on the hair, and eco-sustainability of the end product.

All the formulations containing MFC according to the present invention proved to remain stable for at least 90 days in the various conditions tested. Furthermore, the addition of MFC makes it possible to maintain the desired viscous consistency (dense emulsion), which at the same time being formulations that can still be easily processed. Without being tied down to any theory, it is believed that the good processability and stability of the formulations are linked to the three-dimensional lattice formed by the MFC contained therein, which delays the coalescence of the dispersed phase and imparts thixotropic properties / shear-thinning effect on the compound; as a result, the MFC dispersion appears relatively viscous in static conditions, whereas it has a good flowability (it becomes less dense, less viscous) when it is shaken, stirred or subjected to stresses; i.e., the viscosity of an MFC dispersion is reduced when shear forces are applied. As a result, the viscosity at rest, η₀, is relatively high, whereas the viscosity at high values of shear stress drops accordingly. This result, together with others, is hence attributed to the microfibrillated cellulose, which has a good suspending and stabilising capacity in the stationary state, while at the same time renders the dispersion easy to process, for example using it as a spray. For the same reason, following upon application, the formulations containing MFC do not trickle and do not drip, thanks to a high value of viscosity at rest, η₀, guaranteed by the presence of MFC in the formulation, this making it possible to obtain stable applications, unlike what is obtained using the control formulations.

It has moreover been discovered that the use of the MFC dispersion in the aforementioned formulations for hair treatment enables an even distribution thereof on the hair by virtue of the already described rheological properties. Moreover, a continuous and homogeneous film is formed along the fibre of the hair that does not lead to the sensation of heaviness typical of leave-on products. The film thus obtained by application of the formulation adheres sufficiently to the hair during drying and moreover imparts on the hair a series of properties: protection from external mechanical agents (rubbing, draughts), protection from humidity and heat, reduction of friction during mechanical treatment of the hair (conditioning effect) both on wet hair and on dry hair, longer-lasting styling during the operations of hair styling and strengthening effect.

### Detailed description

### A. Definitions

The term "hair" refers to one or more locks of hair, as well as to natural components of hair. The term "hair" likewise refers to natural or treated hair, for example hair that has been exposed to formulations for destructuring.

"Effective amount" refers to an amount of an additive that, when applied as part of a desired dosage, renders the suspension stable for a given period of time.

The terms "pharmaceutically acceptable" and "cosmetically acceptable" are used in an interchangeable way and refer to those compounds, materials, compositions, and/or forms of dosage that are, in the context of a solid medical evaluation, suitable for use in contact with tissues of human beings and animals, without excessive toxicity, irritation, allergic response, or other problems or complications, commensurate with a reasonable risk/benefit ratio. More specifically, "pharmaceutically acceptable" refers to a material, compound, or composition suitable for use in contact with the hair. Pharmaceutically acceptable ingredients are known to persons skilled in the art.

As used herein, the term "shampoo" in general refers to a liquid, solid, or semi-solid formulation applied on the hair, which contains a detergent or soap for washing hair. As used herein, "balm or conditioner" in general refers to a formulation (for example, a liquid, cream, lotion, gel, semi-solid and solid) applied on hair and then rinsed off (i.e., of the rinse-off type) or left on the hair without rinsing (i.e., of the leave-on type) in order to soften the hair, smooth it, and/or change the shine or lustre thereof.

As used herein, the term "composition for dyeing hair" in general refers to a product (liquid, semi-solid, cream), applied on the hair and then rinsed off. In some cosmetic forms, the composition for hair dye is constituted by just one dyeing formulation, whereas in other cosmetic forms, like the one described in the present invention, the hair dye is constituted by a dyeing and oxidant formulation, mixed at the moment of use, in order to obtain a change in the shade/reflection of the colour or in the shine or lustre of the hair.

As used herein, the term "dyeing formulation" refers to an oil-on-water (O/W) emulsion, in the form of liquid, gel, or cream, which may contain pigments of various nature and other additives.

As used herein, the term "oxidant formulation" refers to a solution, dispersion, or O/W emulsion in the form of liquid, gel or cream that may contain an oxidant and other additives.

The terms "suspending agent" or "stabilising agent", which are used herein as synonyms, refer to molecules or polymers that by modifying the viscosity of the formulation, adsorbing at the interface between the dispersed phase and the continuous phase, counter the phenomenon of coalescence of the dispersed phase, with consequent phase separation.

The term "filming agent" refers to polymers that, by adhering to the surface of the hair, and/or skin, leave a layer, also referred to as film, characterised by being continuous, cohesive, and removable; this layer can bestow properties on the substrate on which it is applied.

The term "anti-slip agent" refers to a substance capable of preventing vertical dripping and trickling of a liquid composition from a vertical surface on which it has been applied in a significant amount. For instance, the distance covered on vertical surface following upon the application, in a given time interval, is reduced by more than 5%, 10%, 20%, 30%, 40%, 50%, 60%, 70%, 80%, 90%, 95% or more with respect to the distance covered by a corresponding liquid composition that does not contain the anti-drip. For instance, an effect of "reduction of dripping" exists if the distance covered, following upon application on a vertical cardboard material, is reduced by at least 10% in the first 10 seconds from application as compared to the distance covered by a comparison formulation not containing the anti-drip agent, applied in the same conditions.

By "distance covered" we mean the length of the longest trickle that extends from the area of application of the liquid formulation, measured immediately after application of mechanical stresses and measured from beneath (i.e., from the lower limit of the original area of application) upwards.

The term "microfibrillated cellulose" or "MFC" as used in the context of the present invention, refers to cellulose fibres of any possible origin. In particular, according to the present invention, MFC refers to cellulose in the form of fibres that has been subjected to a mechanical treatment so as to increase the surface area of the aforesaid fibres and reduce their dimensions in terms of cross section and length, where these dimensional reductions lead to fibre diameters of the order of nanometres and lengths of the order of micrometres. MFC (also known as "microfibrillated cellulose", "crosslinked cellulose", "superfine cellulose", "nanofibrillated cellulose", etc.) is prepared from cellulose fibres defibrillated using high pressure, and/or intense mechanical forces. In the starting cellulose material (typically described as "cellulose pulp") the fibres "singulated" or separated are not present or are present in a non-significant or non-observable way. Instead, MFC has singulated fibrils or groups of fibrils that can be easily observed by means of conventional optical spectroscopy. At times these fibrils and groups of fibrils are also described as "(micro)fibrils". According to the present invention, any reference to "fibrils" also includes said groups of fibrils.

### B. Formulations for hair care

The formulations described herein refer to hair care. The formulations may contain one or more stabilisers and/or conditioners and/or filming agents and/or anti-drip agents, which can be combined with one or more pharmaceutically acceptable carriers and/or excipients, which are considered safe and effective on human hair, and/or on the scalp, and can be applied to the hair of an individual, without causing undesired side effects, such as burning, itching and/or redness or similar adverse reactions.

The formulations may moreover contain one or more excipients that can change the pH. The preferred range of pH is between pH 3 and pH 10.

Listed hereinafter are different functional compounds that may be present in the cosmetic formulations for hair care according to the present invention.

### B1. Active agent - MFC

The active agent according to the present invention is constituted by one or more types of microfibrillated cellulose (MFC).

Microfibrillated cellulose is described, for example, in US 4 481 077, US 4 374 702, and US 4 341 807. According to US 4 374 702 ("Turbak"), microfibrillated cellulose has properties distinguishable from those of the types of cellulose previously known. Thanks to its extensive surface area and the high aspect ratio (length-to-diameter ratio), it is believed that microfibrillated cellulose has a good capacity to form rigid lattices. In solution, MFC typically forms dispersions of a viscous-gel type with shear-thinning properties. The large surface area of MFC and the large number of surface hydroxyl groups enable this material to have a high water-retention capacity. MFC, according to "Turbak", is produced by passing a liquid suspension of cellulose through an orifice (i.e., an opening, or valve, or needle) of small diameter, where the suspension is subjected to a high drop in pressure and a high shear velocity, followed by a high-velocity decelerating impact; passage through the orifice is repeated until the cellulose suspension becomes a stable suspension. The process converts cellulose into microfibrillated cellulose without any substantial chemical changes to the starting cellulose material. An improved process for the production of particularly homogeneous MFC is described in WO 2007/091942. The microfibrillated cellulose used in this invention can be produced according to any process comprised in the state of the art. Preferably, said methods include at least one mechanical step and at least one homogenisation step. The step of mechanical pre-treatment chiefly has the purpose of "dismembering" the cellulose pulp so as to increase accessibility to the cell wall, i.e., increase the surface area and thus increase the value of water retention. In the refiner, which is preferably used in the mechanical pre-treatment step, one or two rotating disks are used; i.e., the suspension of cellulose pulp is subjected to shear forces. Prior to the step of mechanical or chemical pre-treatment, or simultaneously with the mechanical or chemical pre-treatment or instead of the mechanical pre-treatment, enzymatic pre-treatment of the cellulose pulp is an additional step that may be preferred for some applications.

Usually, the average length of the cellulose fibrils and of the groups of fibrils is from 100 nm to 50 µm, preferably from 500 nm to 25 µm, more preferably from 1 µm to 10 µm, even more preferably from 3 µm to 10 µm, while the average diameter of the cellulose fibrils and of the groups of fibrils is from 1 nm to 500 nm, preferably from 5 nm to 100 nm, more preferably from 10 nm to 30 nm. Consequently, the average aspect ratio of the cellulose fibrils and of the groups of fibrils is high. The length and diameter of the fibril are determined using Atomic Force Microscopy, and/or Scanning Electron Microscopy. In particular applications, the surface area of MFC is large. In the context of the present invention, the term "surface area" refers to the total surface area of the cellulose material per unit of mass.

Preferably, the microfibrillated cellulose is a dispersion having a viscosity at rest of at least 5000 mPa*s, more preferably at least 10000 mPa*s, even more preferably at least 20000 mPa*s, as measured in water as dispersing medium, and with a solid content of MFC of 2 wt%; the viscosity at rest was measured using the Anton Paar rheometer (with cup-and-bob geometry), and the value obtained was recorded from the linear visco-elastic region of an amplitude scan (frequency 1 Hz).

By "viscosity at rest" (viscosity at zero shear stress) we mean a measurement of the stability of the three-dimensional lattice responsible for formation of the dispersion of a gel type.

The MFC used in this invention preferably possesses improved viscous properties. It is deemed that the three-dimensional lattice of an MFC dispersion is stable, whereas this stability is lost (the dimensional lattice is broken) when the MFC dispersion is subjected to shear forces, for example, when the gel is carried (for example, sprayed or pumped) or the like. On account of the high viscosity at rest, as soon as the material sprayed strikes the surface on which it has been sprayed, the three-dimensional lattice is reformed (i.e., it increases in viscosity), and no dripping is observed.

In particular applications, the microfibrillated cellulose has a water-retention capacity of at least 50%, more preferably at least 60%, even more preferably at least 70%.

The water-retention capacity describes the capacity for the MFC to withhold the water within the structure thereof, and also this property is correlated to the surface area of the MFC. In the context of the present invention, the water-retention capacity is measured by diluting the sample of MFC up to 0.3 wt% in water and then subjecting the sample to centrifuging for 15 minutes at 1000G; the limpid supernatant is then separated, while the precipitate is weighed. The water-retention capacity is given by (mV/mT) - 1, where mV is the weight of the wet precipitate, and mT is the weight of the dried precipitate.

Theoretically, the starting material for the production of MFC may be any material containing cellulose, in particular cellulose originating from wood, annual plants, cotton, linen, straw, sugar cane, some species of algae, jute, beetroot, citrus fruits, waste from the production of paper and the foodstuff industry; or also cellulose of bacterial origin and/or animal origin, for example cellulose from tunicates. In particular applications, materials with a wood base, both hard wood and soft wood, as well as mixtures thereof are used as raw materials. More preferably, soft wood is used as starting material, whether of just one type and as mixtures of a number of types.

The term "MFC", according to the present invention, comprises one or more types of microfibrillated cellulose, with the constraint that no type of MFC has undergone modifications of a chemical nature.

Microfibrillated cellulose, according to the present invention, may be subjected to at least one dehydration and/or drying step. The drying step is preferably chosen from among freeze-drying, spray-drying, roller-drying, drying in a convection oven, flash-drying, or the like. "Never-dried" MFC may also be used, in the present invention, the solid content of the cellulose suspension prior to being added to the hair-treatment formulation may range between 0.1% and 25 wt%.

### B2. Additives

In addition to the active part, the cosmetic formulation of the present invention may contain other additives (from 0.05 wt% to 90 wt%) normally used in cosmetic formulations for hair treatment, i.e., one or more cosmetically acceptable excipients, where the one or more excipients are selected from the group constituted by water, surfactants, vitamins, natural extracts, preservatives, chelating agents, perfumes, oxidants, antioxidants, hair-dyeing agents, proteins, amino acids, humectants, emollients, penetrating agents, setting agents for hair, emulsifiers, opacifiers, propellants, carriers, salts, pH regulators, neutralisation agents, buffers, hair conditioners, anti-static agents, anti-frizz agents, anti-dandruff agents, and corresponding mixtures.

### In particular:

### B21. Excipients

The formulations usually contain one or more cosmetically acceptable excipients. Cosmetically acceptable excipients include, but are not limited to, water, preservatives, antioxidants, chelating agents, sunscreening agents, vitamins, tints, hair-dyeing agents, proteins, amino acids, natural extracts such as plant extracts, humectants, fragrances, perfumes, oils, emollients, lubricants, butters, penetrating agents, rheology modifiers, polymers, resins, hair fixatives, filming agents, surfactants, detergents, emulsifiers, opacifiers, oxidants, stabilisers, volatile agents, propellants, liquid vehicles, carriers, salts, pH regulators (for example, citric acid), neutralising agents, buffers, anti-static agents, anti-frizz agents, anti-dandruff agents, absorbents, and corresponding combinations thereof. In some forms, the formulations contain the active agent, water, and at least two or more cosmetically acceptable excipients, amongst which, optionally, a preservative and/or a fragrance.

### B22. Surfactants

Surfactants are surface-active agents that are able to reduce the surface tension of water and cause the formulation to slide through the hair. Surfactants likewise include detergents and soaps. Surfactants may be non-ionic, amphoteric, anionic, or cationic.

Suitable anionic surfactants include, but are not limited to, those containing carboxylate, sulphonate and sulphate ions. Examples of anionic surfactants include: sodium, potassium, ammonium alkyl sulphonates and long-chain alkyl aryl sulphonates, such as sodium or ammonium dodecylbenzene sulphonate; sodium dialkyl sulphosuccinates, such as sodium bis-(2-ethylthioxyl)-sulphosuccinate; alkyl sulphates, such as sodium lauryl sulphate, ammonium myreth sulphate, ammonium myristyl sulphate, and ammonium stearate.

Cationic surfactants include, but are not limited to, quaternary-ammonium compounds such as benzalkonium chloride, benzethonium chloride, cetrimonium bromide, stearyl dimethylbenzyl ammonium chloride, and polyoxyethylene coconut amine. Examples of non-ionic surfactants include ethylene glycol monostearate, propylene glycol myristate, glyceryl monostearate, glyceryl stearate, polyglyceryl-4 oleate, acylated sorbitan, acylated saccharose, PEG-150 laurate, PEG-400 monolaurate, polyoxyethylene monolaurate, polysorbates, polyoxyethylene octylfenylether, PEG-1000 cetyl ether, polyoxyethylene tridecyl ether, polypropylene glycol butyl ether, Poloxamer 401, stearoyl monoisopropanolamide, and tallow amide hydrogenated in polyoxyethylene.

Examples of amphoteric surfactants include sodium N-dodecyl-beta-alanine, sodium N-lauryl-β-iminodipropionate, myristo amphoacetate, lauryl betaine, and lauryl sulphobetaine. More than one surfactant may be included in the formulation.

The surfactants are optionally included in an amount ranging from approximately 0.01 wt% to approximately 15 wt% of the formulation, preferably from approximately 1 wt% to approximately 18 wt% of the formulation.

### B23. Emollients

"Emollient" refers to a material that protects from humidity or irritation, softens, soothes, coats, lubricates, hydrates, protects from and/or cleans the skin. Emollients suitable for use in the formulation include, but are not limited to, a silicone compound (for example, dimethicone, cyclomethicone, dimethicone copolyol or a mixture of cyclopentasiloxane and cyclopentasiloxane polysilicone), polyols such as sorbitol, glycerin, propylene glycol, ethylene glycol, polyethylene glycol, caprilyl glycol, polypropylene glycol, 1.3-butanediol, hexylene glycol, isoprene glycol, xylitol; ethylhexyl palmitate; a triglyceride such as caprylic/capric triglyceride, and fatty-acid ethers or esters such as cetearyl isononanoate or cetyl palmitate. More than one emollient may be included in the formulation.

The emollient is optionally included in an amount ranging from approximately 0.05 wt% to approximately 10 wt% of the formulation, preferably from approximately 0.1 wt% to approximately 3 wt% of the formulation.

### B24. Emulsifiers

The formulations may moreover contain one or more emulsifiers. Suitable emulsifiers include, but are not limited to, copolymers of an unsaturated ester and a styrene-sulphonate monomer, cetearyl alcohol, glyceryl ester, polyoxyethylene glycol ether of cetearyl alcohol, stearic acid, polyglycerol ethers or esters, polysorbate-20, ceteareth-20, lecithin, glycol stearate, polysorbate-60, or polysorbate-80, or corresponding combinations. More than one emulsifier may be included in the formulation.

The emulsifier is optionally included in an amount ranging from approximately 0.05 wt% to approximately 15 wt% of the formulation, preferably from approximately 0.1 wt% to approximately 3 wt% of the formulation.

### B25. Preservatives

One or more preservatives may be included in the formulations to prevent microbial growth in the formulations. Suitable preservatives include, but are not limited to, compounds containing glycerin (for example, glycerin or ethylhexylglycerin or phenoxyethanol), benzyl alcohol, parabens (methylparaben, ethylparaben, propylparaben, butylparaben, isobutylparaben, etc.), sodium benzoate, ethylenediaminetetraacetic acid (EDTA), potassium sorbate and/or extract of grapefruit seeds, or corresponding combinations. More than one preservative may be included in the formulation. Other preservatives are known in the cosmetic industries and include salicylic acid, DMDM hydantoin, formaldehyde, chlorophenesin, triclosan, imidazolidinyl urea, diazolidinyl urea, sorbic acid, methylisothiazolinone, sodium dehydroacete, dehydroacetic acid, quaternium-15, stearalkonium chloride, zinc pyrithione, sodium metabisulphite, 2-bromo-2-nitropropane, chlorhexidine digluconate, polyaminopropyl biguanide, benzalkonium chloride, sodium sulphite, sodium salicylate, citric acid, neem oil, essential oils of various type, lactic acid, and vitamin E (tocopherol). The preservative is optionally included in an amount ranging from approximately 0.01 wt% to approximately 5 wt% of the formulation, preferably from approximately 0.3 wt% to approximately 2 wt% of the formulation.

Preferably, the formulations are without parabens.

### B26. Conditioning agents (conditioners)

One or more conditioning agents may be included in the formulations. Suitable conditioning agents include, but are not limited to, cetrimonium chloride, polymeric quaternary-ammonium salts of hydroxyethyl cellulose (HEC) reacted with trimethyl ammonium substituted epoxide (Polyquaternium-10), silicone-based agents (for example, silicone quaternium-8), panthenol, hydrolized wheat and/or soy protein, amino acids (for example, wheat amino acids), rice bran wax, Brassica derivatives, *Limnanthes alba* seed oil, mango oil, grapeseed oil, jojoba seed oil, sweet-almond oil, hydroxyethyl behenamidopropyl dimonium chloride, aloe-leaf extract, aloe-vera juice, phytantriol, panthenol, retinyl palmitate, behentrimonium methosulphate, cyclopentasiloxane, quaternium-91, stearamidopropyl dimethylamine, and corresponding combinations. The conditioning agent may be included in an amount ranging from approximately 0.01 wt% to approximately 5 wt% of the formulation, preferably from approximately 0.3 wt% to approximately 3 wt% of the formulation.

### B27. Diluents

"Diluent", as used herein, refers to a substance that dilutes the active ingredient and the other additives present in the formulations. Water is the preferred diluent. The formulations usually contain water in an amount higher than 1 wt%, preferably higher than 5 wt%, more preferably higher than 50 wt%, and most preferably higher than 80 wt%. Alcohols, such as ethyl alcohol and isopropyl alcohol, may be used at low concentrations (approximately 0.5 wt% of the formulation) to enhance penetration in the hair, and/or reduce the smell.

### B28. Rheology modifiers

The formulations according to the present invention may contain one or more viscosity modifying agents, for example agents that increase viscosity. Classes of said agents include, but are not limited to, viscous liquids, in particular hydrated natural polymers. The aforesaid viscosity modifying agents are well known to persons skilled in the cosmetic sector.

### B29. Antioxidants

The formulations of the present invention may contain one or more antioxidants. Examples include, but are not limited to, tocopherols, BHT, ascorbic acid, *Camellia sinensis* leaf extract, ascorbyl palmitate, magnesium ascorbyl phosphate, carotenoids, resveratrol, triethyl citrate, arbutin, kojic acid, tetrahexyldecyl ascorbate, superoxide dismutase, zinc, sodium metabisulphite, licopene, and corresponding combinations.

### B30. Opacifiers

The formulations of the present invention may also contain one or more opacifiers, at times used to render the formulations opaque.

### B31. Oxidants

The formulations of the present invention may also contain one or more oxidants. Examples include, but are not limited to, hydrogen peroxide and persulphates used for their capacity for oxidation of natural and/or artificial pigments.

### B32. Consistency factors

The formulations of the present invention may contain one or more consistency factors, which are additives having multiple function: filming function, hydro-repellent function, stabilising function, structuring function, and viscosity-modifying function. They also present protective and emollient properties and affect the flowability and rheology of the end product.

The consistency factors are generally esters of fatty acids, of natural or synthetic origin. Some examples comprise, but are not limited to: cetyl alcohol, cetostearyl alcohol, behenyl alcohol, and waxes.

### B33. Dyes - pigments

The formulations may contain one or more pigments, i.e., chemical compounds capable of producing, in given conditions, visible colour in certain materials. These compounds are divided into oxidation pigments (permanent pigments) and direct pigments (temporary, semi-permanent, pigments); some examples of oxidation dyes comprise, but are not limited to: diamines, aminophenols, phenols, naphthols, and resorcinols. Some examples of direct pigments comprise: conjugated derivatives of diazene, derivatives of triphenylmethane, conjugated derivatives of hydrazine, indoamines, indophenols, aromatic amines, aminophenols, and nitrophenols. The dyes may be included in an amount ranging from approximately 0 wt% to approximately 20 wt% of the formulation, preferably from approximately 0 wt% to approximately 5 wt% of the formulation.

### B34. Suspending agents

The formulations of the present invention may contain one or more suspending agents, i.e., chemical compounds of a prevalently polymeric nature able to favour dispersion of particles and reduce sedimentation thereof when added to fluid formulations. Examples of suspending agents are well known to persons skilled in the sector and include, but are not limited to, polymeric quaternary-ammonium salts derived from ethyl methacrylate, i.e., poly-(2-(methacryloxy)-ethyl)-trimethylammonium chloride (Polyquaternium-37), carrageenan, guar gum, locust-bean gum, xanthan gum, and cellulose ether.

The cosmetic formulation for hair care of the present invention may be present in formulas of different types. Examples are provided hereinafter.

### • Shampoo

The cosmetic formulation for hair care may be in the form of a shampoo. The shampoo contains MFC in a concentration ranging from 0.1 wt% t₀ 5 wt% by weight, preferably from 0.1 wt% to 3 wt% by weight. In addition, the shampoo may include from approximately 0.5 wt% to approximately 18 wt% of surfactant materials. Surfactants used in the compositions for shampoo are known to persons skilled in the art and are described, for example, in the patents US 6,706,258 and US 7,598,213.

### • Leave-on hair-conditioning balm

The cosmetic formulation for hair care may be in the form of a leave-on hair-conditioning balm. In addition, the leave-on hair-conditioning balm may include surfactants, humectants, plant extracts with emollient function, consistency factors, preservatives, chelating agents, and pH correctors. The formulation may comprise other synthetic or natural polymers or polymers deriving from biological preparation processes, which are functionalised, where appropriate, for example with cationic or neutral groups. These polymers may have a stabilising or strengthening action on the compositions, and/or a conditioning action (deposition on the surface of the skin or of the hair).

The leave-on hair-conditioning balm contains the MFC agent in a concentration ranging from 0.1 wt% to 5 wt%, more preferably from 0.1 wt% to 3 wt%.

### • Product for dyeing hair

A product for dyeing hair is provided, constituted by two formulations, i.e., a first dyeing formulation that is mixed at the moment of use with a second oxidising formulation. For dyeing applications, a sufficient amount of dyeing formulation is mixed with a sufficient amount of oxidant formulation prior to application on the hair. The products for dyeing hair may thus be described as follows.

### - Dyeing formulation

In this embodiment, the dyeing formulation contains the MFC agent in a concentration ranging from 0.1 wt% to 5 wt% by weight, preferably from 0.1 wt% to 3 wt% by weight, in a suitable carrier, usually a diluent, preferably water.

The dyeing formulation may contain, in addition, from 5 wt% to 15 wt% of surfactant materials and may also contain emulsifiers, pH correctors, buffers, antioxidants, and chelating agents. The dyeing formulation may contain permanent pigments, semi-permanent pigments, and demi-permanent pigments, by oxidation or by direct dye, of synthetic and/or natural origin, in a concentration from 0 wt% to 20 wt%.

### - Oxidant formulation

The oxidant formulation may contain any concentration of MFC contains the MFC agent in a concentration ranging from 0.1 wt% to 5 wt% by weight, preferably from 0.1 wt% to 3 wt% by weight, in a suitable carrier, usually a diluent, preferably water.

The oxidant formulation may contain, in addition, an oxidant, a chelating agent, and a pH corrector.

A further aspect of the present invention regards a process for preparing a cosmetic composition for hair care, which comprises mixing at least one oil phase and at least one water phase in this way to form an oil-in-water (O/W) emulsion, said at least one oil phase or said at least one water phase comprising at least one microfibrillated cellulose (MFC), which is characterised by a water-retention capacity of at least 60%, preferably of at least 70%.

In conclusion, the present invention has shown that MFC is able to function as stabiliser of the specific formulations for hair care, moreover presenting an increased conditioning and strengthening effect and long-lasting styling. Furthermore, it can effectively replace synthetic polymers normally used in cosmetics, such as (co)polyacrylate (carbopol, polyquaternium-37) and polymeric quaternary-ammonium salts.

The aims previously set have been achieved thanks to the characteristics of the present invention. It has been discovered that the addition of microfibrillated cellulose (MFC) to the present cosmetic formulations for hair care improves stability thereof, enabling substitution of acrylic polymers and ammonium salts from traditional formulations; the addition of MFC moreover adds a conditioning and strengthening effect and an effect of long-lasting styling of the hair on which they are applied.

The examples described below are provided for a better understanding of the present invention.

### Examples

For all the formulations 2-wt% water dispersions of microfibrillated cellulose were used.

The weight concentration of MFC in the end product was 0.5 wt% for the formulations shampoo and leave-on hair-conditioning product, whereas, as regards the composition for dyeing hair, the concentration was 0.5 wt% for the dyeing formulation and 0.75 wt% for the oxidant formulation; all the products were obtained following the "New developments procedure" of Davines S.p.A. The average length of the cellulose fibrils used in the formulations listed hereinafter ranged from 3 µm to 10 µm, the average diameter of the cellulose fibrils and of the groups of fibrils ranged from 10 nm to 30 nm; the length and diameter of each fibril were determined using Atomic-Force Microscopy and/or Scanning Electron Microscopy.

The microfibrillated cellulose used in the examples provided hereinafter was a dispersion having a viscosity at rest of at least 20000 mPa*s as measured in water as dispersing medium and with a solid content of MFC of 2 wt%; the viscosity at rest was measured using the Anton Paar rheometer (with cup-and-bob geometry); the value obtained was recorded from the linear visco-elastic region of an amplitude scan (frequency 1 Hz). The microfibrillated cellulose used in the examples provided below had a water-retention capacity of at least 70%, measured by diluting the sample of MFC up to 0.3 wt% in water and then subjecting the sample to centrifuging for 15 minutes at 1000G; the limpid supernatant was then separated, whereas the precipitate was weighed. The water-retention capacity was obtained as (mV/mT) - 1 where mV is the weight of the wet precipitate and mT is the weight of the dried precipitate.

### • A) Shampoo

The combability of hair treated with formulations containing MFC (A), without MFC (B), with different (co)polyacrylate polymers (C, D) was tested; provided in *Table 1* are the examples of formulation.

The stability of the same formulations in relation to the phenomenon of coalescence of the emulsion was also tested.

**Table 1: Examples of shampoo formulation.**

| **Position** | **Function** | **Composition** | **Producer** | **A (%)** | **B (%)** | **C (%)** | **D (%)** |
|---|---|---|---|---|---|---|---|
| 1 | Diluent | Water | | q.s. to 100 | q.s. to 100 | q.s. to 100 | q.s. to 100 |
| 2 | Active agent | Cellulose | BORREGAARD | 0.5 | 0 | 0 | 0 |
| 3 | Suspending agent | Acrylates/C10-30 alkyl acrylate crosspolymer | LUBRIZOL | 0 | 0 | 0.5 | 0 |
| 4 | Suspending agent | Carbomer | LUBRIZOL | 0 | 0 | 0 | 0.5 |
| 5 | Humectant | Glycerin | | 4 | 4 | 4 | 4 |
| 6 | Rheology modifier | Xanthan gum | CPKELPO | 0.15 | 0.15 | 0.15 | 0.15 |
| 7 | Rheology modifier | Sclerotium gum | CARGILL | 0.05 | 0.05 | 0.05 | 0.05 |
| 8 | Emollient | Dicaprylyl ether & Lauryl alcohol | BASF | 0.3 | 0.3 | 0.3 | 0.3 |
| 9 | Consistency factor | Cetearyl alcohol | SABO | 1 | 1 | 1 | 1 |
| 10 | Consistency factor | Cetyl alcohol | SABO | 0.2 | 0.2 | 0.2 | 0.2 |
| 11 | Conditioning agent | Brassicyl isoleucinate esylate & Brassica alcohol | INOLEX | 3 | 3 | 3 | 3 |
| 12 | Anionic surfactant | Sodium lauroyl methyl isethionate & Sodium methyl isethionate | INNOSPEC | 2.7 | 2.7 | 2.7 | 2.7 |
| 13 | Anionic surfactant | Sodium cocoyl alaninate | SINO LION USA | 9.1 | 9.1 | 9.1 | 9.1 |
| 14 | Non-ionic surfactant | Decyl glucoside | BASF | 3.6 | 3.6 | 3.6 | 3.6 |
| 15 | Non-ionic | Lauryl | BASF | 1 | 1 | 1 | 1 |
| | surfactant | glucoside | | | | | |
| 16 | Anionic surfactant | Sodium lauroyl glutamate | ZSCHIMMER & SCHWARZ | 2.4 | 2.4 | 2.4 | 2.4 |
| 17 | pH modifier | Lactic acid | | 1 | 1 | 1 | 1 |
| 18 | Perfume | Perfume | GRC PARFUM | 0.5 | 0.5 | 0.5 | 0.5 |
| 19 | Preservative | Benzyl alcohol | DEKABEN | 0.5 | 0.5 | 0.5 | 0.5 |

### o Formulation procedure

1. The active agent or the suspending agent (see corresponding *Table 1*) and the rheology modifiers together with the humectant are dispersed in the diluent by means of mechanical stirring.
2. All the surfactants and emollients are added to the previous dispersion one at a time.
3. The phase thus obtained is heated and stirred.
4. Once a temperature of 35°C is reached, preservatives, perfume, and pH correctors are added.

### Results

### Stability tests

In order to carry out the stability tests on the formulations, two parallel approaches were used:
- The LUMiFuge^{®} instrumentation was used, which enables correlation of a possible variation of transmittance, measured on the length of the cuvette used for the test (containing the formulation to be analysed), with effects of instability therein.
- Empirical tests of conservation of the formulations were conducted in different conditions.

Given in *Table 2* are the results of the stability tests carried out via instrumentation LUMiFuge^{®}. The LUMiFuge^{®} analysis was chosen since it is predictive of an index of chemico-physical stability, given that its result is expressed by a phase-separation index associated with phenomena of instability, this being demonstrated from the literature that can be found in various fields, in addition to the cosmetic field, for example in the foodstuff and pharmaceutical sectors. As operating conditions (test duration, temperature, and centrifugation speed) the following values were, respectively, chosen: 3h, 45°C, 2000 rpm. These conditions were chosen and calibrated so as to highlight differences of stability between the formulations analysed.

Appearing in *Table 3* are the results of the empirical stability tests, in different conditions (4°C, 45°C, room temperature, 53°C, and thermal cycle).

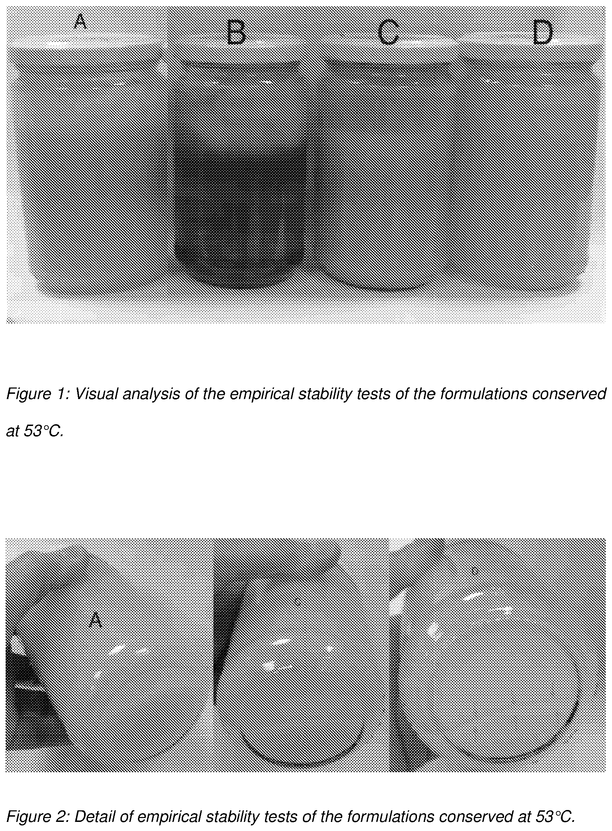

*Figures 1* and *2* show the photographs associated with the empirical stability tests.

**Table 2: Analysis of stability formulations using the LUMiFuge^{®} instrumentation. The spectra shown give the percentage transmittance on the axis of the ordinates and the position in the cuvette on the axis of the abscissae.**

| **formulation** | **spectrum** |
|---|---|
| A | 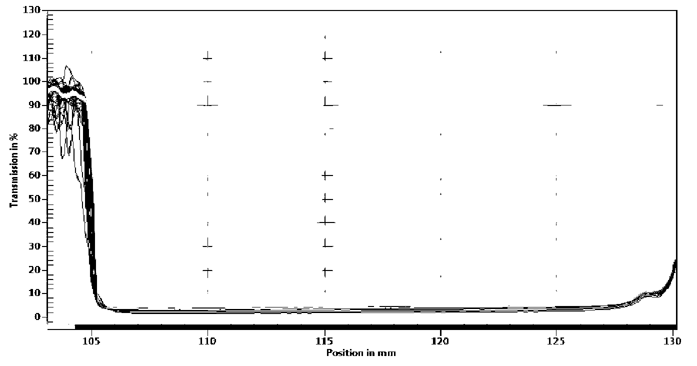 |
| B | 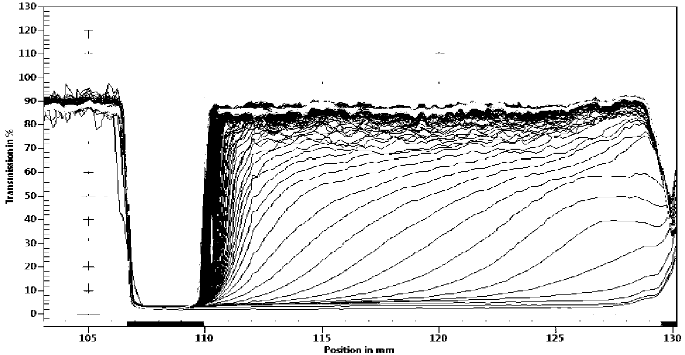 |
| C | 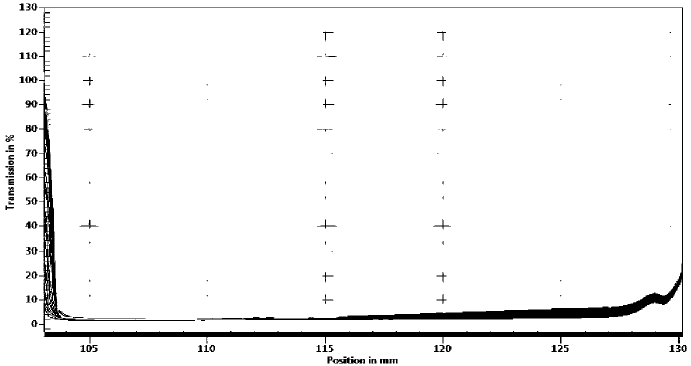 |
| D | 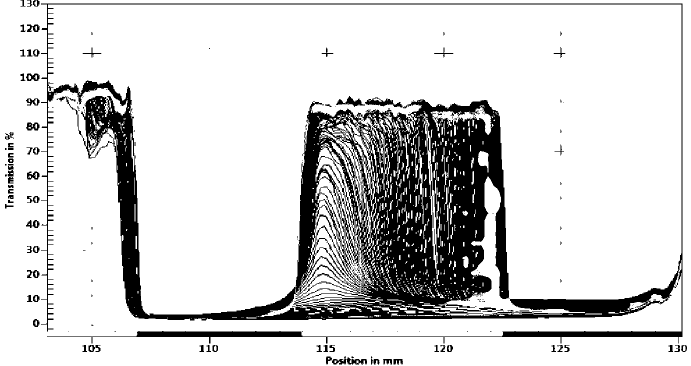 |

**Table 3: Results of empirical tests of conservation of the formulations.**

| **Formulations** | **Room temperature** | **45°C** | **53°C** | **Thermal cycle** | **4°C** |
|---|---|---|---|---|---|
| A | no separation | no separation | no separation | no separation | no separation |
| B | complete separation | complete separation | complete separation | complete separation | complete separation |
| C | complete separation | no separation | slight separation on the bottom of the container | no separation | no separation |
| D | no separation | separation | slight separation on the bottom of the container | no separation | no separation |

### ∘ Conclusions

From the tests conducted on the samples examined in both the empirical tests and the instrumental analysis it may be noted how the formulation (B) in absence of MFC is less stable than the formulation (A) containing MFC. In addition, the suspending power of MFC is decidedly improved as compared to the formulation containing Carbomer and, from the empirical tests, also slightly improved as compared to the suspending agent Acrylates/C10-30 alkyl acrylate crosspolymer tested. This shows the possibility of using MFC as alternative to suspending agents of a synthetic nature.

### Combability tests

To carry out the combability tests it was decided to test only the formulations of examples A and C, i.e., the samples that are suitable from the standpoint of stability of the formulation, given that B and D highlighted a high index of separation under LUMiFuge^{®} analysis; these formulations were tested using as reference a "placebo" shampoo formulation for the statistical analysis of the results, the formula and formulation procedure of which are provided in *Table 4.*

As substrate for the tests, level-5 locks of human hair were used, standard as regards weight and width. These locks were first subjected to a first treatment of bleaching so as to obtain destructuring of the hair fibres, a step that makes it possible to highlight more clearly differences in the effectiveness of the formulations used for the tests. The locks were then subjected to a preparation procedure and finally tested using dedicated instrumentation.

**Table 4: "Placebo" shampoo formulation.**

| **Position** | **Function** | **Composition** | **Producer** | **%** |
|---|---|---|---|---|
| 1 | Diluent | Water | | q.s. to 100 |
| 2 | Humectant | Glycerin | | 4 |
| 3 | Rheology modifier | Sclerotium gum | | 0.15 |
| 4 | Emollient | Dicaprylyl ether & Lauryl alcohol | BASF | 0.3 |
| 5 | Consistency factor | Cetearyl alcohol | SABO | 1 |
| 6 | Consistency factor | Cetyl alcohol | SABO | 0.2 |
| 7 | Natural conditioner | Brassicyl isoleucinate esylate & Brassica alcohol | INNOSPEC | 3 |
| 8 | Anionic surfactant | Sodium lauroyl methyl isethionate & Sodium methyl isethionate | INNOSPEC | 2.7 |
| 9 | Anionic surfactant | Sodium cocoyl alaninate | SINO LION USA | 9.1 |
| 10 | Non-ionic surfactant | Decyl glucoside | BASF | 3.6 |
| 11 | Non-ionic surfactant | Lauryl glucoside | BASF | 1 |
| 12 | Anionic surfactant | Sodium lauroyl glutamate | ZSCHIMMER & SCHWARZ | 2.4 |
| 13 | pH modifier | Lactic acid | | 1 |
| 14 | Perfume | Perfume | GRC PARFUM | 0.5 |
| 15 | Preservative | Benzyl alcohol | DEKABEN | 0.5 |

### o Formulation procedure

See formulation procedure described previously.

Description of combability tests
- Bleaching
   1. The bleaching mixture was prepared using 6 g per lock of Davines Mask Hair Bleaching Powder^{®} in mixture with Davines Activator 40vol^{®} in a weight ratio of 1:2.
   2. The bleaching mixture was applied over the entire length of the lock, front and back, and the lock was wrapped in a silver-paper wrapper; the treatment was applied for 50 min.
   3. At the end of the treatment the lock was washed using Davines Hair Care SOLU^{®} shampoo, rinsed, and then the conditioner Well Being^{®} of Davines Hair care was applied. It was left on for 5 minutes, and then the lock was rinsed and finally dried.
- Procedure for combability tests
   1. The dried lock was weighed.
   2. The dried lock was wetted under running water.
   3. The wet lock was blotted using a paper cloth for 30 seconds.
   4. The damp lock was weighed to determine the weight of the water absorbed and to check that it was uniform for all the samples; in the case where this condition was not met, water was sprayed on the lock so as to render the samples uniform.
   5. An amount of 0.4 g of formulation was applied on the damp lock, half on one side and half on the other side, and the treatment was spread manually over the entire lock.
   6. The treated lock was washed under running water for 30 seconds and blotted using a paper cloth for 30 seconds.
   7. The damp treated lock was combed 10 times with a narrow-toothed comb and 10 times with a wide-toothed comb.
   8. The combability analysis was conducted on the wet hair using the instrumentation *Texture Analysis Plus (Extended Height)* equipped with a comb having teeth arranged in four rows, provided along with the instrument.
   9. The lock was dried using a hair-dryer.
   10. The lock was combed 10 times with a wide-toothed comb and 10 times with a narrow-toothed comb.
   11. The combability analysis was conducted on the dry hair using the instrumentation *Texture Analysis Plus (Extended Height)* equipped with a comb having teeth arranged in four rows, provided along with the instrument.
- Combability tests

The instrumental analyses were carried out using three locks of hair for each example of formulation tested; each individual lock was analysed using the instrument carrying out 20 combing actions. The instrument returns, as output, a graph that gives the force expressed by the dynamometer as a function of the position of the comb, for each individual combing action.

From the average of the values obtained on the three locks of hair analysed for each treatment, combability is inversely proportional to the value of work expressed by the graph. The dried lock treated with the formulation A presented lower values of work. Use of MFC renders the fibres more untanglable as compared to synthetic polymers.

The data provided by the instrument are available on demand at the cosmetics firm Davines S.p.A.

From the graphs provided by the instrument, analysis of the data was conducted in the following way:
1. The value of work expressed by the dynamometer for each individual combing action carried out was extrapolated; the combability is inversely proportional to the value of work expressed by the instrument.
2. The average of the work expressed by the dynamometer was computed over the 20 combing actions performed.
3. The general average was computed considering the average of the values previously obtained on the three locks representative of the treatment analysed.
4. The significance of the results obtained was analysed using the statistical analysis method by sets of numbers that do not show a normal distribution, the Kruskal-Wallis test.

### ∘ Results

The results of the analyses of the combability data are provided in *Graphs 1* and 2.

*Graph 1: Results of the combability tests on dry hair. The combability is inversely proportional to the combing force: the lower the combing force, the more the lock is combable.*

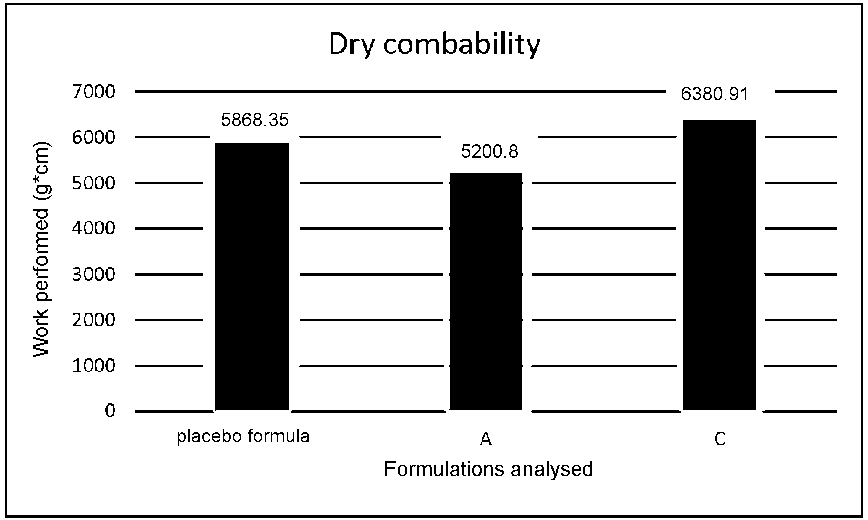

The combability data on the dried locks highlight that the formula with MFC (sample A) is significantly superior both to the formula with synthetic polymer (sample C) and to the placebo formula.

*Graph 2: Results of the combability tests on wet hair. The combability is inversely proportional to the combing force: the lower the combing force, the more the lock is combable.*

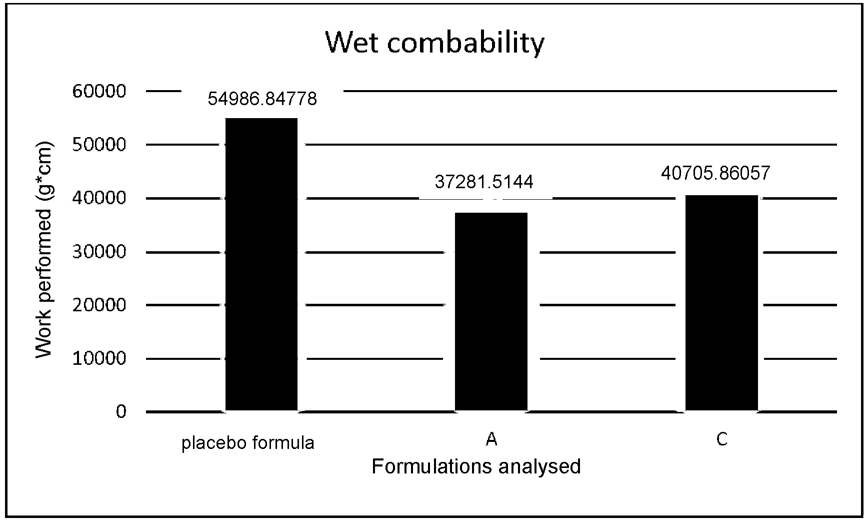

The combability data on the wet locks highlight that the sample with MFC (sample A) is significantly positive as compared to the placebo and superior to the synthetic polymer (sample C).

### ∘ Conclusions

From the tests carried out it emerges that, when MFC is introduced into a shampoo formulation, it certainly brings about an increase in combability as compared to a formulation without MFC and also presents improved results in terms of combability, in particular on dry hair as compared to the formula containing the synthetic polymer (acrylates/C10-30 alkyl acrylate crosspolymer).

These data prove, once more, the possibility of replacing synthetic polymers with MFC due to its capacity for providing high performance in terms of combability (conditioning effect).

### Tests of foam-stabilisation booster effect

To test the foaming effect generated by MFC, it was decided to test two formulations prepared *ad hoc* for this test, the components of which are provided in *Table 5*; given the simplicity of the formulations tested, the preparation procedure is not provided.

It was chosen to use as reference for this type of tests the surfactant present in the formula with highest concentration.

Then, the formulation in the presence of MFC at 0.5 wt% in aqueous solvent (A') was compared with the aqueous solution without MFC (B').

**Table 5: Formulas used in the tests of the stabilising effect on foam.**

| **Material** | **Composition** | **Producer** | **A' (%)** | **B' (%)** |
|---|---|---|---|---|
| Diluent | Water | | q.s. to 100 | q.s. to 100 |
| Anionic surfactant | Sodium cocoyl alaninate | SINO LION USA | 9.1 | 9.1 |
| Active agent | Cellulose | BORREGAARD | 0.5 | 0 |

### o Test procedure

The test was carried out as follows:
1. The sample to be tested was prepared by setting 0.5 ml of formulation A' or B' in a graduated cylinder and adding deionised water so as to obtain a 0.5% concentration of formulation to be tested.
2. The two cylinders were shaken manually 15 times.
3. The height of the front of the foam was recorded at t₀.
4. The height of the front of the foam was recorded after 15 minutes.

### o Results

From the tests carried out, it may be noted that the sample containing MFC (A') at t₀ showed a height of the front of the foam of 9.1 cm; the sample without MFC (B') showed, instead, a height of the foam of 8.4 cm.

The datum was then detected after 15', and, notwithstanding the fact that the numeric value of the height of the front of the foam had not varied, it may be noted that the sample without MFC (B') showed a less stable foam.

### ∘ Conclusions

The tests prove how the addition of MFC in the formulation can have a function of foam-stabilising booster.

### Final conclusions

As regards the shampoo formulation it was found that the sample containing MFC was significantly better than the formulas containing synthetic polymers (Carbomer or acrylates/C10-30 alkyl acrylate crosspolymer) both from the standpoint of stability and from the standpoint of combability; in the case of MFC, the foam-stabilising booster property was moreover found.

These results prove, in the case of the formulation of a shampoo type, the possible use, as an alternative to the synthetic polymers, of MFC as cosmetic additive with multiple functions: suspending agent, stabiliser, foam-stabilising booster, and conditioner.

### • B) Leave-on hair-conditioning balm

Testing was carried out of combability of hair treated with formulations containing MFC (A), without MFC (B), with synthetic polymers, namely: Polyquaternium-37 (C), guar hydroxypropyltrimonium chloride (D), and Polyquaternium-10 (E). Appearing in *Table* 6 are the examples of formulation.

The stability in time and in the various conditions of the formulations described previously was tested in relation to the phenomenon of coalescence of the emulsion; furthermore, the styling-lasting effect and the strengthening effect on hair were tested.

**Table 6: Examples of formulation of leave-on hair-conditioning balm.**

| **Position** | **Function** | **Composition** | **Producer** | **A (%)** | **B (%)** | **C (%)** | **D (%)** | **E (%)** |
|---|---|---|---|---|---|---|---|---|
| 1 | Diluent | Water | | q.s. to 100 | q.s. to 100 | q.s. to 100 | q.s. to 100 | q.s. to 100 |
| 2 | Active agent | Cellulose | BORREGAARD | 0.5 | 0 | 0 | 0 | 0 |
| 3 | Suspending agent | Polyquaternium-37 | BASF | 0 | 0 | 0.5 | 0 | 0 |
| 4 | Suspending agent | Guar hydroxypropyltrimonium chloride | SOLVAY | 0 | 0 | 0 | 0.5 | 0 |
| 5 | Conditioning agent | Polyquaternium-10 | DOW CHEMICAL | 0 | 0 | 0 | 0 | 0.5 |
| 6 | Consistency factor | Cetearyl alcohol | SABO | 1 | 1 | 1 | 1 | 1 |
| 7 | Consistency factor | Cetyl alcohol | SABO | 0.4 | 0.4 | 0.4 | 0.4 | 0.4 |
| 8 | Emollient | Poyglyceryl-4 oleate & Glyceryl olivate & Hydrogenated rapeseed alcohol | GILAS | 0.1 | 0.1 | 0.1 | 0.1 | 0.1 |
| 9 | Emollient | Phenethyl benzoate | ASHLAND INDUSTRIES | 0.12 | 0.12 | 0.12 | 0.12 | 0.12 |
| 10 | Consistency factor | Dicocoyl pentaerythrityl | BASF | 0.2 | 0.2 | 0.2 | 0.2 | 0.2 |
| 11 | Emulsifier | Cetearyl olivate & Sorbitan olivate | HALLSTAR | 2.75 | 2.75 | 2.75 | 2.75 | 2.75 |
| 12 | Diluent | Water | DAVINES | 2 | 2 | 2 | 2 | 2 |
| 13 | Chelating agent | Disodium EDTA | | 0.1 | 0.1 | 0.1 | 0.1 | 0.1 |
| 14 | Preservative | Sodium benzoate | | 0.19 | 0.19 | 0.19 | 0.19 | 0.19 |
| 15 | Preservative | Benzyl alcohol | DEKABEN | 0.7 | 0.7 | 0.7 | 0.7 | 0.7 |
| 16 | Perfume | Perfume | GRC PARFUM | 0.2 | 0.2 | 0.2 | 0.2 | 0.2 |
| 17 | pH modifier | Lactic acid | | 0.15 | 0.15 | 0.15 | 0.15 | 0.15 |

∘ Formulation procedure
   1. The active agent or the suspending agents or the conditioners (see corresponding table) are dispersed in the diluent and mechanically stirred.
   2. Chelating agents and preservatives are added and stirred.
   3. The mixture thus obtained is heated to 80°C, and then emulsifiers, consistency factors, and the emollient are added.
   4. The product is emulsified, left to cool, and finally perfume is added at room temperature.
∘ Results

### Stability tests

To carry out the tests of stability of the formulations the same approaches used for the previous example were followed.

The formulation of example D was not analysed because it presented phase separation immediately after the emulsifying process.

*Table 7* below provides the results of the stability tests carried out using LUMiFuge^{®} instrumentation. As operating conditions, namely, test duration, temperature, and centrifugation speed, the values 12h, 30°C, 4000 rpm were respectively chosen; these conditions were chosen so as to highlight differences in stability between the formulations analysed.

*Table 8* provides the results of the empirical stability tests, in different conditions (4°C, 45°C, room temperature, 53°C, and thermal cycle).

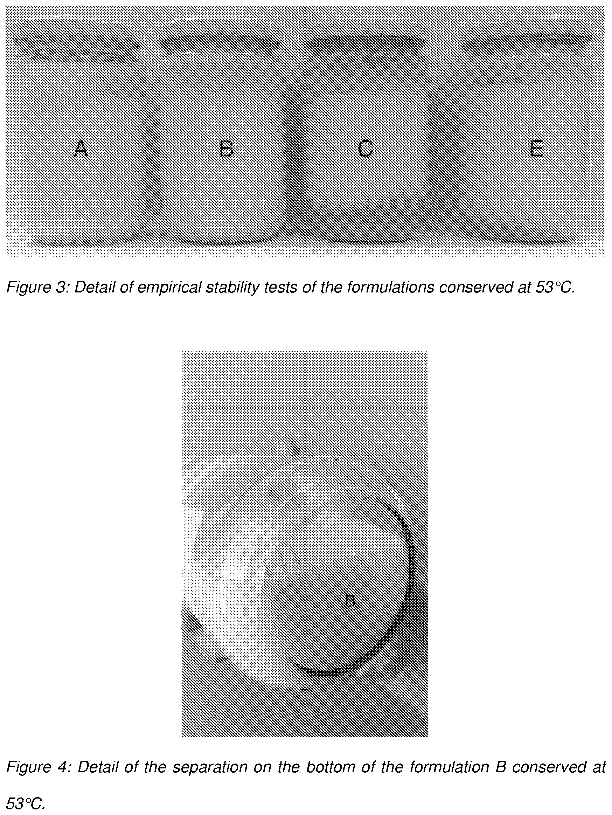

*Figures 3* and *4* show the photographs associated with the empirical stability tests.

**Table 7: Analysis of stability of formulations using the LUMiFuge^{®} instrumentation. The spectra shown give the percentage transmittance on the axis of the ordinates and the position in the cuvette on the axis of the abscissae.**

| **formulation** | **spectrum** |
|---|---|
| A | 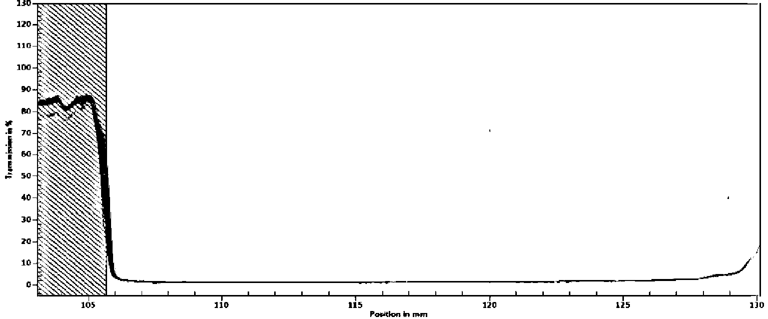 |
| B | 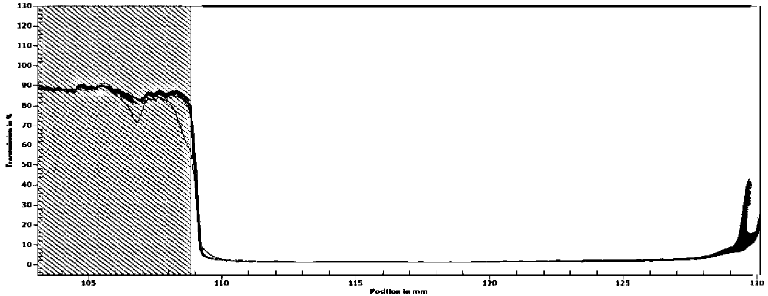 |
| C | 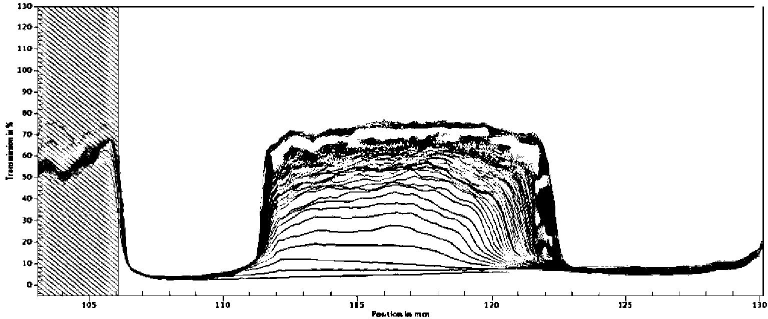 |
| E | 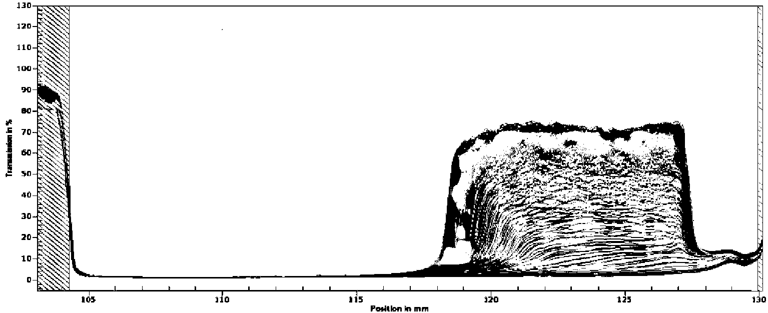 |

**Table 8: Results of empirical tests of conservation of the formulations.**

| **Formulations** | **Room temperature** | **45°C** | **53°C** | **Thermal cycle** | **4°C** |
|---|---|---|---|---|---|
| A | no separation | no separation | no separation | no separation | no separation |
| B | no separation | no separation | start of separation | no separation | no separation |
| C | complete separation | complete separation | complete separation | no separation | no separation |
| E | no separation | complete separation | partial separation on bottom of container | no separation | no separation |

### o Conclusions

From the tests mentioned above, it emerges that the formulation with MFC (A) is stable both under the instrumental analysis and under the visual analysis of the empirical tests, unlike similar formulations where MFC was replaced with synthetic polymers (C, D, and E) and unlike the formulation not containing MFC (B), where a start of separation (represented in detail in *Figure 4*) was, however, noted.

It follows that, with the introduction of MFC in the formulations, it is possible to replace successfully the synthetic polymers (such as polyquaternium-37), in the case where it is intended to obtain the suspending and stabilising effect, which is necessary for structuring the cosmetic product.

### Combability tests

To carry out the combability tests, it was decided to test the formulations of examples A, B, C, and E, i.e., the samples that are suitable from the standpoint of stability.

The formulations A, C, and E were tested in comparison with the formulation B used as "placebo" for the statistical analysis of the results. As substrate for the tests, level-5 locks of natural hair were used, standard as regards weight and width: these locks were first subjected to two bleaching treatments, in order to highlight differences in the effectiveness of the formulations used for the tests. The locks were then subjected to a preparation procedure and finally tested using dedicated instrumentation.

### ∘ Combability test procedure

- Bleaching
   See bleaching procedure of previous example.
- Combability test procedure
   1. The dried lock was weighed.
   2. The dried lock was wetted under running water.
   3. The wet lock was blotted using a paper cloth for 30 seconds.
   4. The damp lock was weighed to determine the weight of the water absorbed and to check that it was uniform for all the samples; in the case where this condition was not met, water was sprayed on the lock so as to render the samples uniform.
   5. An amount of 0.5 g of formulation was applied on the damp lock, half on one side and half on the other side, and the treatment was spread manually over the entire extension of the lock.
   6. The damp treated lock was combed 10 times with a wide-toothed comb and 10 times with a narrow-toothed comb.
   7. Analysis of combability on wet hair was carried out using the instrumentation *Texture Analysis Plus (Extended Height)* equipped with a comb having teeth arranged in four rows, provided along with the instrument.
   8. The lock was dried using a hair-dryer and simultaneously combed with a wide-toothed comb.
   9. The dried treated lock was combed just once before the test was carried out.
   10. The analysis of combability on dry hair was carried out using the instrumentation *Texture Analysis Plus (Extended Height)* equipped with a comb having teeth arranged in four rows, provided along with the instrument.
- Combability tests

See combability test of the previous example.

The results of the analyses of the combability data appear in *Graph 3.*

### o Results

*Graph 3: Results of the combability tests on dry hair.*

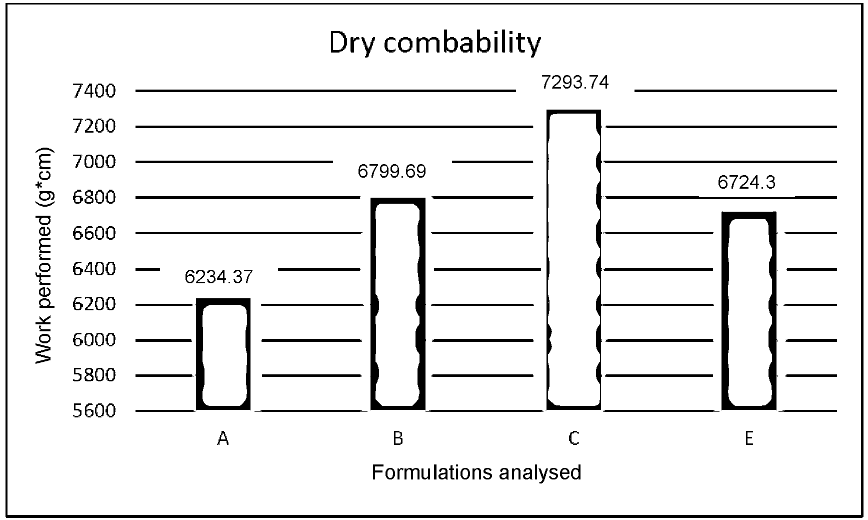

**The sample** A containing MFC gave a result of combability significantly superior to both the sample B, without MFC, and the sample containing synthetic polymer (C). The sample A containing MFC was slightly improved as compared to the sample E.

### ∘ Conclusions

As highlighted for the shampoo formulation, also for the formulation of the leave-on hair-conditioning balm the effectiveness of MFC of acting positively on hair combability was demonstrated. In fact, from the tests carried out, the sample containing MFC presented values of combability on dry hair better than the formula with polyquaternium-37 and superior to the results of the formula containing polyquanternium-10.

These data once again prove the possibility of replacing the synthetic and partially natural polymers, which present high performance in terms of combability (conditioning effect), with MFC.

### Tests on styling-lasting effect

To carry out the tests on the styling-lasting effect, the formulations A, B, and C were analysed so as to compare the action of MFC with that of the formulation containing synthetic polymer (polyquaternium-37) and with that of formulation not containing any polymer. The tests were conducted using level-5 locks of natural hair, standard as regards weight and length. The effect of *curl retention* (*C*.*R*.) in different time intervals, i.e., the effect of retention of the curl on the treated locks, was evaluated on these locks by applying the following equation: $C . R . \left(%\right) = \frac{I - {I}_{t}}{I - {I}_{0}} ⋅ 100$ where I is the length of the extended lock, I₀ is the length of the curled lock at the initial instant, and It is the length of the lock at instant t.

It follows that the higher the value of C.R., the higher the capacity for long-lasting styling of the product tested.
∘ Procedure
   1. The lock was wetted under running water.
   2. The lock was blotted using a paper cloth for 30 seconds.
   3. An amount of 0.5 g of formulation was applied on the lock, half on each side, and spread out manually.
   4. The styling was applied on the lock with the aid of a hair curler and left to dry in an oven at 45°C for 24 h.
   5. The lock was released from the hair curler and the length at instant t₀ was measured.
   6. The treated lock was put in an oven at 40°C with a humidity of 70% for a pre-set time interval.
   7. The extension undergone by the lock was measured, and the value of C.R. (%) was calculated.
∘ Results

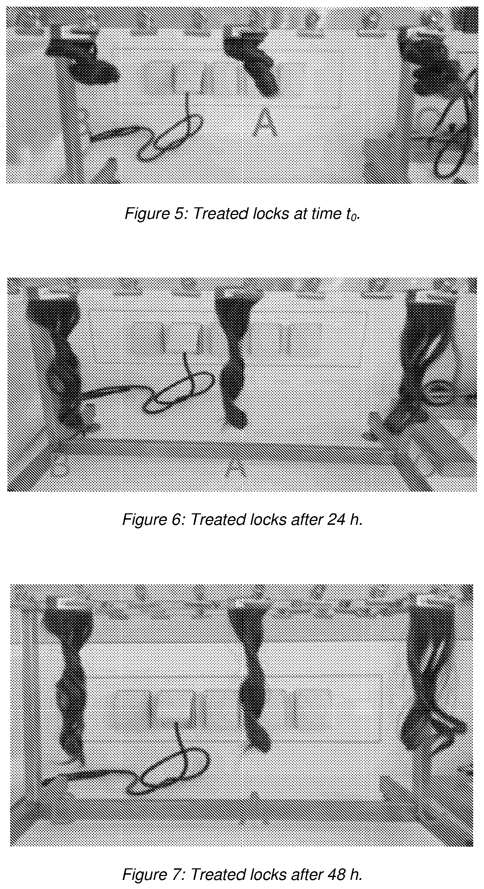

*Figures 5,* 6, and 7 show the photographs of the locks analysed at instant t₀, after 24 h, and after 48 h.

From the evaluations of C.R. (%) appearing in *Table 9*, it is evident how the formulation containing MFC (A) presents a longer-lasting styling effect as compared to the formulation not containing MFC (B) and to the formulation containing synthetic polymer (C).

**Table 9: Results of the analyses of C.R. (%).**

| | **C.R.(%)** | | |
|---|---|---|---|
| **Time** | **A** | **B** | **C** |
| 15 min | 82.22 | 77.66 | 81.21 |
| 24 h | 32.22 | 23.4 | 24.31 |
| 48 h | 26.11 | 23.34 | 17.68 |

### ∘ Conclusions

The lock treated with the formulation A presents C.R. data that are markedly improved as compared to the other formulations, which demonstrates that the formulation A presents a longer-lasting styling effect that is witnessed also by the visual appearance of the curl, which retains over time a better definition as compared to the curls obtained with the formulations B and C, respectively without MFC and without MFC but containing synthetic polymer.

### Ultimate-strength tests

To carry out the ultimate-strength tests it was decided to test the formulation of sample A so as to detect the protective properties of MFC; this formulation was tested against the "placebo" formulation for statistical analysis of the results, this "placebo" formulation corresponding to sample B, without MFC. As substrate for the tests, level-5 locks of natural hair were used, standard as regards weight and width: these locks were first subjected to two bleaching treatments, in order to highlight differences in the effectiveness of the formulations used for the tests. The locks then underwent a preparatory procedure and were finally tested using dedicated instrumentation.
- Ultimate-strength test procedure
- Bleaching

See bleaching procedure of previous tests.
- Ultimate-strength test procedure
   1. The dried lock was weighed.
   2. The dried lock was wetted under running water.
   3. The wet lock was blotted using a paper cloth for 30 seconds.
   4. The damp lock was weighed to determine the weight of the water absorbed and to check that it was uniform for all the samples; in the case where this condition was not met, water was sprayed on the lock so as to render the samples uniform.
   5. An amount of 0.5 g of formulation was applied on the damp lock, half on one side and half on the other side, and the treatment was spread manually over the entire extension of the lock.
   6. The lock was dried using a hair-dryer and simultaneously combed with a wide-toothed comb.
   7. The dried treated lock was straightened using the *Exalta hair straightener EL-201.*
   8. Points 2, 3, and 4 were repeated.
   9. Analysis of the ultimate strength on wet hair was carried out using the instrumentation *Texture Analysis Plus (Extended Height)* equipped with pneumatic clamps for tensile tests, provided along with the instrument.
- Ultimate-strength tests

The instrumental analyses were conducted using one lock of hair per example of formulation tested; from the individual lock, 20 hairs having a length 10 cm were analysed; the rate of sliding of the instrument was constant at 0.5 mm/s. The instrument yields at output the ultimate strength i.e., the tensile force necessary to lead to failure of the hair.

The data produced by the instrument are available on demand at the cosmetics firm Davines S.p.A.

From the graphs produced by the instrument the data were analysed in the following way:
1. The value of ultimate strength measured by the dynamometer for each individual hair analysed was extrapolated.
2. The average of the values obtained on the 20 hairs analysed was computed.
3. The significance of the results obtained was analysed using the two-tailed Student's t test for independent samples.

The results of the analyses of the ultimate-strength data appear in *Graph 4.*

### o Results

*Graph 4* shows the value of ultimate strength of the lock treated with the formulation containing MFC, where this value is higher than in the case of the lock treated with sample B.

*Graph 4: Ultimate strength of hair treated with leave-on hair-conditioning balm containing MFC (A) and not containing MFC (B).*

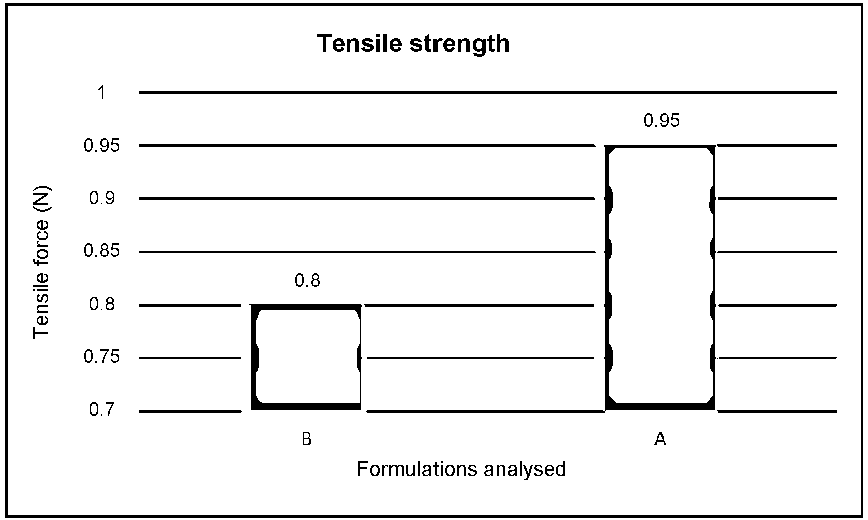

### ∘ Conclusions

As regards the results of the ultimate-strength tests, it may be inferred that the lock treated with the formulation containing MFC (sample A) is significantly stronger than the one treated with the formulation without MFC (sample B). This effect can be correlated to the filming action of MFC, which, by coating the hair and adhering thereto, increases the ultimate strength thereof.

### Final conclusions

As regards the formulation of a leave-on hair-conditioning balm, it was found that the sample treated with MFC was significantly superior to the formulas containing synthetic polymers (polyquaternium-37, guar hydroxypropyltrimonium chloride) and superior also to the formulas containing polymeric quaternary-ammonium salts of hydroxyethyl cellulose (HEC) reacted with trimethylammonium-substituted epoxide (polyquaternium-10), both from the standpoint of stability and as regards combability.

Use of MFC showed strengthening properties on hair, as well as an improved effect in terms of lasting styling. These properties are particularly sought after in a cosmetic end product designed for hair care.

These results prove, in the case of the formulation of a leave-on hair-conditioning balm, the possible use, as substitution for both synthetic polymers and partially natural quaternised polymers, of MFC as cosmetic additive with multiple functions: suspending agent, stabiliser, conditioner, and strengthener.

### C) Hair-dyeing composition

A composition for dyeing hair was tested consisting of two formulations: a dyeing formulation and an oxidant formulation.

As regards the stability tests, the dyeing and oxidant formulations were tested separately. As regards, instead, the combability tests, the ultimate-strength tests, and the rheological tests, it was decided to test the overall formulation, obtained by mixing in equal parts the dyeing and oxidant formulations, as described in the Davines application manual so as to reproduce the real modalities of application of this type of product, once introduced on the market.

### Oxidant formulation

In this embodiment, the oxidant formulation may contain any concentration of active agent in a suitable carrier, usually a diluent. The oxidant formulation may in addition contain an oxidant.

### Dyeing formulation

In this embodiment, the dyeing formulation may contain any concentration of active agent in a suitable carrier, usually a diluent. The dyeing formulation may moreover contain surfactants, as well as emulsifiers, pH correctors, buffers, antioxidants, and chelating agents. The dyeing formulation may contain permanent, semi-permanent, and demi-permanent pigments of the oxidation or direct-dye type, of synthetic and/or natural origin, in concentrations of from 0 wt% to 20 wt%.

### Overall formulation

For the applications, prior to use, a sufficient amount of dyeing formulation is mixed with a sufficient amount of oxidant formulation, as envisaged in the Davines application manual.

### Dyeing formulations

The capacity for MFC to improve the stability of the dyeing formulation was tested. In particular formulations containing MFC (A) were tested and compared against the same formulation without MFC (B). Appearing in *Table* 10 are the examples of dyeing formulation.

The chemico-physical stability of both of the formulations was tested in different temperature conditions (4°C, 45°C, room temperature - TA, 53°C, and thermal cycle - CT).

**Table 10: Examples of dyeing formulation tested.**

| **Position** | **Material** | **Composition** | **Producer** | **A (%)** | **B (%)** |
|---|---|---|---|---|---|
| 1 | Diluent | Water | | q.s. to 100 | q.s. to 100 |
| 2 | Active agent | Cellulose | BORREGAARD | 0.5 | 0 |
| 3 | pH modifier | Phosphoric acid | | 1.125 | 1.125 |
| 4 | pH modifier | Ethanol amine | | 1.13 | 1.13 |
| 6 | Antioxidant | Sodium sulphite | | 0.2 | 0.2 |
| 7 | Chelating agent | Disodium EDTA | | 0.2 | 0.2 |
| 8 | Non-ionic surfactant | Coco-glucoside | BASF | 4.95 | 4.95 |
| 9 | Amphoteric surfactant | Coco-betaine | ZSCHIMMER & SCHWARZ | 1.665 | 1.665 |
| 10 | Anionic surfactant | Sodium cocoyl-alaninate | SINO LION USA | 2.4 | 2.4 |
| 11 | Humectant | Propylene glycol | OLEON N.W. | 5 | 5 |
| 12 | Emulsifier | Olive oil polyglycerol-6 esters | NATURALIS SRL | 5 | 5 |
| 13 | Consistency factor | Lauryl alcohol & Myristyl alcohol | ZSCHIMMER & SCHWARZ | 4 | 4 |
| 14 | Diluent | Denatured alcohol | SILCOMPA | 15 | 15 |
| 15 | Perfume | Perfume | GRC PARFUM | 0.2 | 0.2 |
| 16 | Dyeing pigments | | | 0-20% | 0-20% |

∘ Formulation procedure
   1. The active agent and/or the antioxidant and the chelating agent (see the corresponding table) are dispersed in the diluent and mechanically stirred.
   2. The pH modifiers and surfactants and, where present, the pigments are added under stirring. The mixture is heated to 75°C and emulsified.
   3. The emulsion thus obtained is cooled to room temperature, and the following are added under stirring: humectant, emulsifier, consistency factor, and perfume.
∘ Results

### Stability tests

Stability was evaluated only through the empirical tests of conservation of the formulations in various conditions of temperature in so far as this type of formulation has a semi-transparent appearance; hence, it is impossible to glean stability data via analysis of the transmittance using the LUMiFuge^{®} instrumentation.

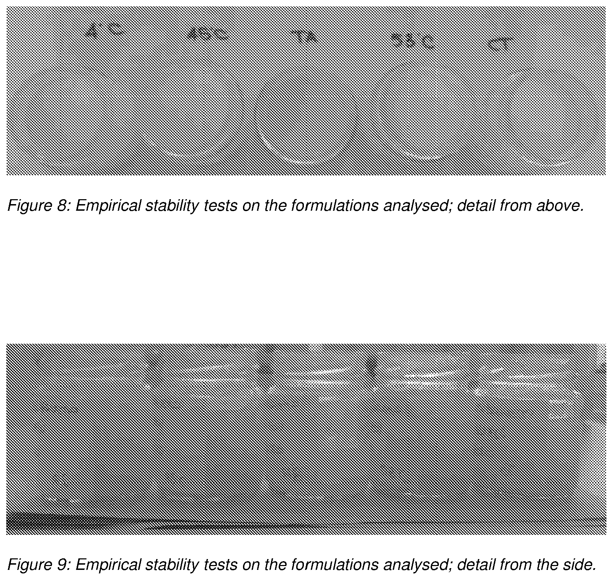

*Figures 8* and *9* show the photographs associated with the empirical stability tests on the formulation A.

In *Table 11,* only the result of the formulation A is given since the product B presents immediate instability after the production process.

**Table 11: Analysis of stability of formulation A.**

| **Formulation** | **Room temperature** | **45°C** | **53°C** | **Thermal cycle** | **4°C** |
|---|---|---|---|---|---|
| A | no separation | no separation | no separation | no separation | no separation |
| B | / | / | / | / | / |

The formulation B, without MFC, could be stabilised by making corrections to the percentages of the raw materials present in the formula. This would, however, entail an increase in the percentage of the non-ionic surfactant and of the emulsifier. The former, in the reaction of production of the raw material, has a portion of petrochemical derivation, whilst the increase of the amount of emulsifier, of natural origin, would entail an increase in the production costs of the end product.

### ∘ Conclusions

From an analysis of stability of the formulation described above, it emerges that MFC guarantees a good stability in the various conditions. A similar formulation, but without MFC, separates markedly after just a few hours from the production process.

The presence of MFC guarantees greater naturalness of the formulation with more contained target cost.

### Oxidant formulation

The capacity for the MFC to improve the stability of the oxidant formulation was tested; in particular, the formulation containing MFC (A) was tested and compared against the same formulation without MFC (B). Appearing in *Table 12* are the examples of formulation.

The chemico-physical stability of both of the formulations was tested in different temperature conditions (room temperature - TA, 45°C).

**Table 12: Examples of oxidant formulation tested.**

| **Position** | **Material** | **Composition** | **Producer** | **A (%)** | **B (%)** |
|---|---|---|---|---|---|
| 1 | Diluent | Water | | q.s. to 100 | q.s. to 100 |
| 2 | Active agent | Cellulose | BORREGAARD | 0.75 | 0 |
| 3 | Oxidant | Hydrogen peroxide | EVONIK | 17.2 | 17.2 |
| 4 | pH modifier | Disodium phosphate | | 0.4 | 0.4 |
| 5 | pH modifier | Phosphoric acid | | 0.2 | 0.2 |
| 6 | Stabiliser | Iron hydroxide | BOZZETTO GROUP | 0.1 | 0.1 |

∘ Formulation procedure
   1. The active agent is dispersed in part of the diluent and mechanically stirred.
   2. pH modifiers and stabilisers are dispersed in the remainder of the diluent and stirred mechanically, and then the oxidant is added.
   3. The phase containing the oxidant is added slowly to the phase containing the active agent, and the mixture produced is stirred mechanically.
∘ Results

### Stability tests

To carry out the stability tests, it was decided to evaluate only the empirical tests of conservation of the formulations in the conditions of room temperature and at 45°C since the evaluation of stability was not made in terms of phase separation but in terms of increase in volume due to the development of oxygen. This phenomenon, when present, is due to the destabilisation of hydrogen peroxide and is highlighted by a significant increase of air bubbles in the formulation that leads to an increase in the level of product in the container used that can be easily identified and measured. Hence, in this case, the analysis of the transmittance using the LUMiFuge^{®} instrumentation is unsuited to obtain stability data.

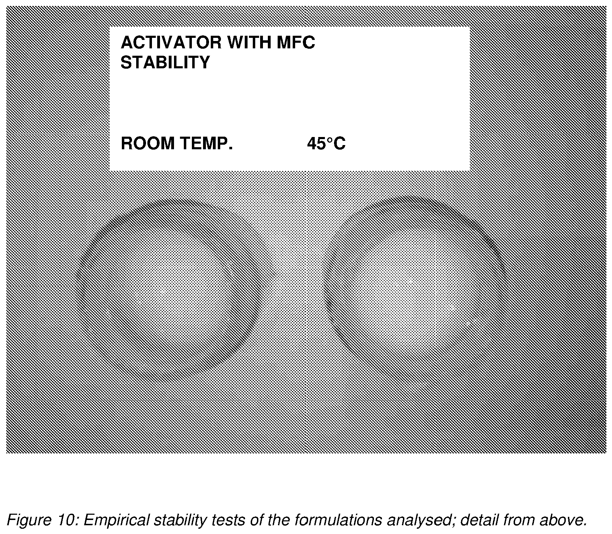

Represented in *Figure 10* is the photograph associated with the empirical stability tests on the formulation A. It was not possible to provide the photographs associated with the formulation B in so far as the latter becomes unstable shortly after its preparation.

### ∘ Conclusions

From an analysis of the stability of the formulation described above, it emerges that MFC guarantees full compatibility even when it is used in association with hydrogen peroxide, which is notoriously a very unstable raw material. MFC can hence be used for creating stable oxidant formulations that can be used in the cosmetic field.

The similar formulation, but without MFC, develops oxygen already just a few hours after the production process, this being a clear sign of instability of the formula.

### Overall hair-dye formulation

To evaluate the strengthening, conditioning, and anti-slip properties of MFC in this type of product, it was decided to test in all cases the overall formulation, i.e., a mixture 1:1 in weight between the dyeing formulation and the oxidant formulation. MFC was introduced into the formulas both in the dyeing formulation and in the oxidant formulation to test the strengthening properties (ultimate-strength test), whereas MFC was inserted only into the dyeing formulation to test the conditioning and anti-slip properties.

In order to carry out the ultimate-strength tests, the dyeing formulation A (with MFC), mixed at the moment of use in equal parts with the oxidant formulation A (with MFC), was tested and compared with the overall Davines commercial formulation (reference formulation), obtained by mixing in equal parts at the moment of use, the Davines dyeing formulation View Gloss^{®} with the Davines oxidant formulation Activator 10vol^{®}.

As regards the combability and anti-slip tests, it was decided to test the dyeing formulation A (with MFC) mixed at the moment of use in equal parts with the commercial oxidant formulation Davines Activator 10vol^{®}. This mixture was tested and compared with the overall Davines commercial formulation (reference formulation) obtained by mixing in equal parts at the moment of use the dyeing formulation Davines View Gloss^{®} and the oxidant formulation Davines Activator 10vol^{®}, in order to be able to highlight more clearly the conditioning and anti-slip effect bestowed only by the dyeing formulation.

### Ultimate-strength tests

As described above, to carry out the ultimate-strength tests it was decided to test the effect of the presence of MFC both in the dyeing formulation and in the oxidant formulation. For this reason, it was chosen to test, for both, the corresponding formulations of example A (see section Dyeing formulation, *Table 10,* and the section Oxidant formulation, *Table* 12) so as to detect the strengthening property of MFC of the overall formulation; mixing of the two formulations at the moment of use was carried out according to the indications described in the Davines user manual. The above formulation was tested in comparison with the overall Davines commercial formulation obtained by mixing, with the same modalities, of the two Davines commercial products View Gloss^{®} and Activator 10vol^{®}.

As substrate for the tests, level-5 locks of natural hair were used, standard as regards weight and width: these locks were first subjected to two bleaching treatments in order to highlight differences in the effectiveness of the formulations used for the tests. The locks were then subjected to a preparatory procedure and finally tested using dedicated instrumentation.
- Ultimate-strength test procedure
- Bleaching

See bleaching procedure of previous tests.
- Ultimate-strength test procedure
   1. The dried lock was weighed.
   2. The dried lock was wetted under running water.
   3. The wet lock was blotted using a paper cloth for 30 seconds.
   4. The damp lock was weighed to determine the weight of the water absorbed and to check that it was uniform for all the samples; in the case where the condition was not met, water was sprayed on the lock using spray so as to render the samples uniform.
   5. Then, 6 g of dyeing formulation to be tested and 6 g of oxidant formulation were weighed; the mixture obtained was applied on the lock according to the Davines application manual.
   6. The mixture prepared was applied on the damp lock and left there for 20 minutes at room temperature.
   7. The treated lock was then washed under running water up to complete removal of the mixture and was blotted using a paper cloth for 30 seconds.
   8. An analysis of ultimate strength was then performed on the wet hair using the instrumentation *Texture Analysis Plus (Extended Height)* equipped with pneumatic clamps for tensile tests, provided along with the instrument.
- Ultimate-strength tests

See section on ultimate-strength tests of leave-on hair-conditioning product.

The results of the analysis of the ultimate-strength data are provided in *Graph 7.*

### ∘ Results

Highlighted in *Graph 5* are the locks treated with the overall formulation containing MFC (A), which are significantly stronger than those treated with the reference commercial formulation.

*Graph 5: Ultimate strength of hair treated with dyeing formulation containing MFC (A) and reference formulation.*

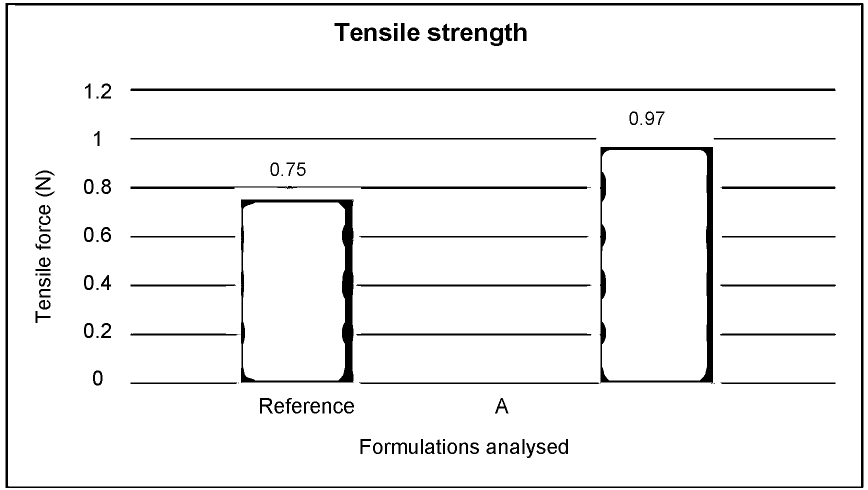

### ∘ Conclusions

As regards the results of ultimate strength it may be inferred that the locks treated with the formulation containing MFC are significantly stronger than those treated with the reference formulation (without MFC). This effect can be correlated to the filming action of MFC, which, by coating the hair and adhering to it, increases the ultimate strength thereof.

### Combability tests

To carry out the combability tests it was decided to test the effect of the presence of MFC in just the dyeing formulation (example A of the *Table 10*). This formulation was tested and compared with the Davines commercial dyeing formulation (reference formulation) View Gloss^{®}.

Both of the dyeing formulations, i.e., those with MFC and the commercial ones, were mixed, prior to application for the test, with equal parts of the oxidant formulation Davines Activator 10vol^{®}, according to the Davines user manual.

As substrate for the tests, level-5 locks of natural hair, standard as regards weight and width, were used: these locks first underwent two bleaching treatments so as to obtain destructuring of the locks in order to highlight differences in the effectiveness of the formulations used for the tests. The locks then underwent a preparatory procedure and were finally tested using dedicated instrumentation.
- Combability-test procedure
- Bleaching

See bleaching procedure of previous tests.
- Combability-test procedure
   1. First 6 g of dyeing formulation and 6 g of commercial activator (Davines Activator 10vol^{®}) were mixed together, and the mixture obtained was then applied on the damp lock and left there for 20 minutes at room temperature.
   2. The treated lock was then washed under running water and blotted using a paper cloth for 30 seconds.
   3. The damp lock was weighed to determine the weight of the water absorbed and to check that it was uniform for all the samples; in the case where this condition was not met, water was sprayed on the lock so as to guarantee uniformity between the samples tested.
   4. The damp treated lock was combed 10 times with a narrow-toothed comb and 10 times with a wide-toothed comb.
   5. Analysis of combability on wet hair was carried out using the instrumentation *Texture Analysis Plus (Extended Height)* equipped with a comb having teeth arranged in four rows, provided along with the instrument.
   6. The lock was dried using a hair-dryer and a wide-toothed comb.
   7. The dried treated lock was combed 10 times with a narrow-toothed comb and 10 times with a wide-toothed comb.
   8. The analysis of combability on dry hair was carried out using the instrumentation *Texture Analysis Plus (Extended Height)* equipped with a comb having teeth arranged in four rows, provided along with the instrument.
- Combability tests

See combability tests of the previous examples; the results of the combability tests are presented in *Graphs 6* and *7.*

### ∘ Results

From *Graphs 6* and *7* it emerges that the dyeing formulation containing MFC showed a better combability, on both dry hair and wet hair, as compared to the formulation without MFC (reference formulation).

*Graphs 6 and 7: Combability is inversely proportional to the combing force: the lower the combing force, the more combable the lock.*

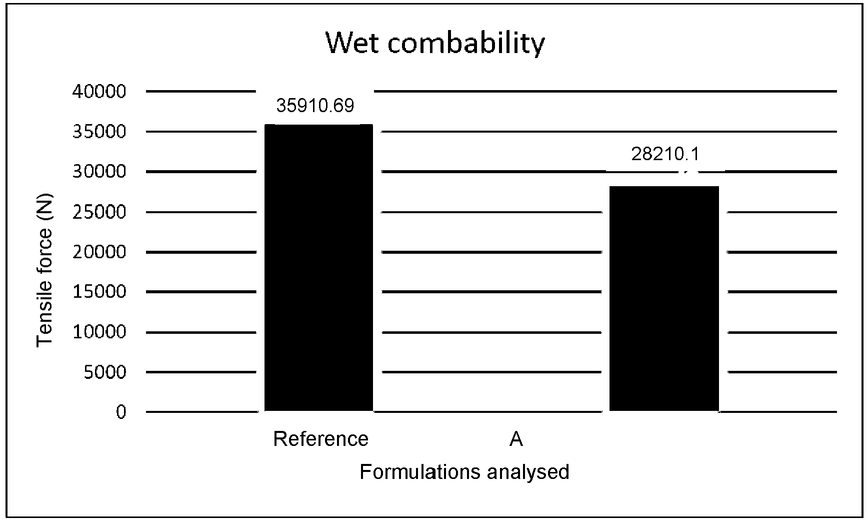

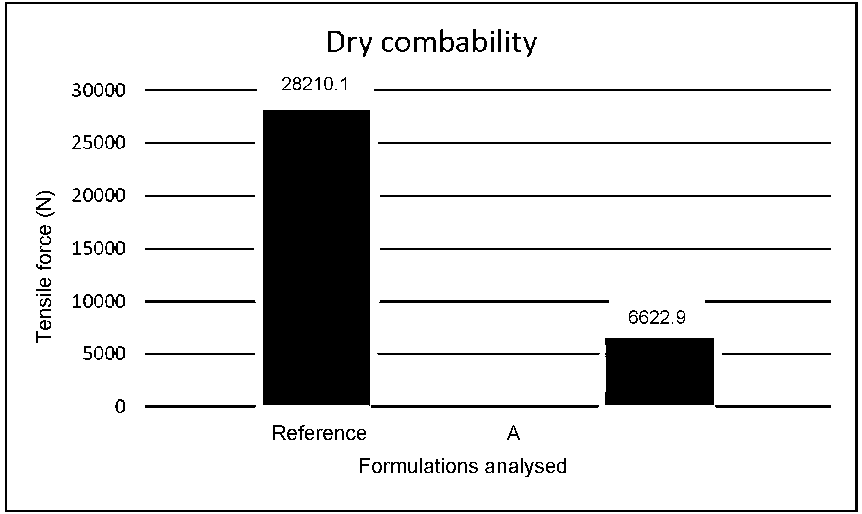

### o Conclusions

The dyeing formulation containing MFC shows a better combability, on both dry hair and wet hair, as compared to similar formulations but without MFC (reference formulation).

### Rheological analysis: anti-slip tests

To carry out the anti-slip tests it was decided to test the effect of introduction of MFC into just the dyeing formulation: the formulation of example A was thus used, in the presence and absence of pigments (see the section Dyeing formulation, *Table 10*) so as to detect the anti-slip properties of MFC.

The dyeing formulations (formulation A without and with pigments) were tested and compared with similar commercial dyeing formulations not containing MFC: Davines View Gloss^{®} (without pigments) and Davines View 6.0^{®} (with pigments).

Both of the dyeing formulations, i.e., those with MFC and the commercial ones, were mixed, prior to application for the test, with equal parts of oxidant formulation Davines Activator 10vol^{®}, as per the Davines user manual.
∘ Anti-slip test procedure
   1. First, 5 g of dyeing formulation to be tested and 5 g of oxidant formulation Davines Activator 10vol^{®} were weighed.
   2. The two formulations of point 1 were mixed together; 1 g of the two formulations were taken and applied at the same height on a panel set in a horizontal position.
   3. The panel was then set in a vertical position, and the distance covered by each individual product in 10 seconds was measured.
∘ Results

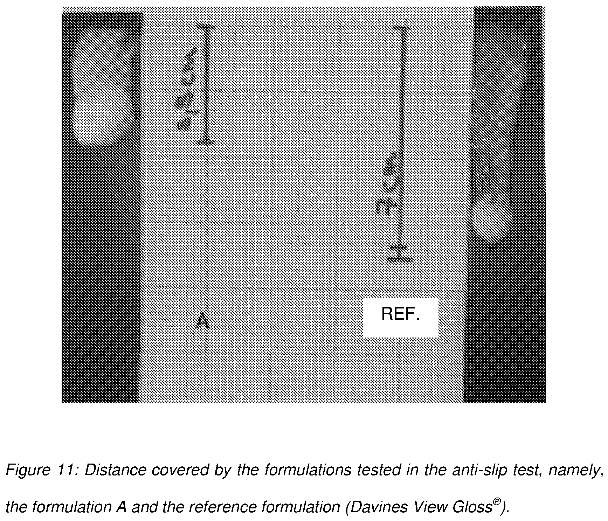

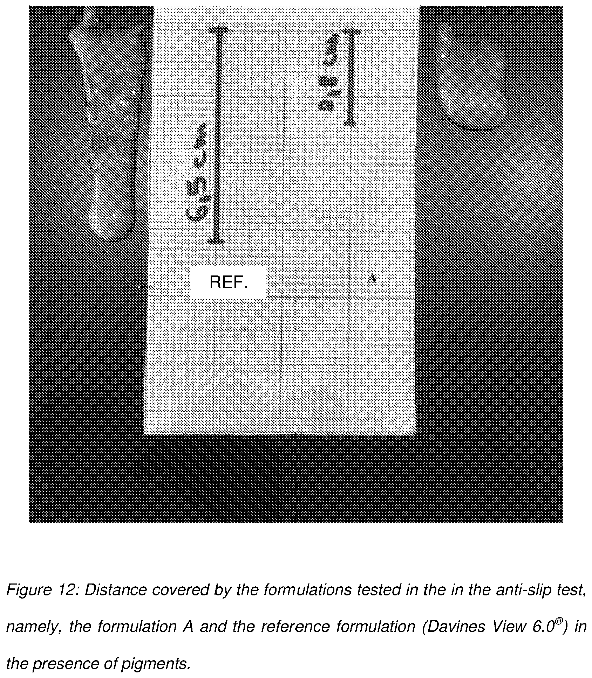

*Figures 11* and *12* show the anti-slip properties of MFC when it is introduced into dyeing formulations, with and without pigments, mixed, at the moment of application in equal parts, with a suitable volume of commercial oxidant formulation Davines Activator 10vol^{®} (reference formulation).

The product containing MFC (A) in 10 seconds flowed along the surface set vertically for a distance of 3.5 cm.

The product not containing MFC (reference formulation) flowed for a distance of 6.8 cm along the same surface.

The addition of MFC, given its thixotropic characteristics, shows a reduction of 48.5% in the distance covered by the formulation.

The product containing MFC (A) in 10 seconds flowed along the surface set vertically for a distance of 2.8 cm.

The product without MFC (reference formulation) flowed for a distance of 6.5 cm along the same surface.

The two formulations contained the same percentage of pigments.

The addition of MFC, given its thixotropic characteristics, showed a reduction of 57% in the distance covered by the formulation.

### ∘ Conclusions

The dyeing formulation containing MFC (A), in the absence of pigments, and/or in the presence of an equal concentration of pigments as compared to the Davines commercial formulation (Davines View 6.0^{®}), showed improved anti-slip properties along a surface set vertically as compared to the similar formulation not containing MFC (reference formulation). This makes it possible to classify MFC as anti-slip agent. In application terms, this results in the possibility of preventing the undesired phenomena of dripping when left on, albeit guaranteeing ease of outflow of the products for dyeing hair from the corresponding containers with the desired viscosity.

### Final conclusions

The inclusion of MFC in the formula of the hair-dyeing product leads to an increase in the stability both as regards the dyeing formulation and as regards the activating formulation. The presence of MFC in the dyeing formulation leads to an increase in the performance of the overall formulation both as regards ultimate strength and combability and as regards the anti-slip capacity of the formulation itself. These results demonstrate the possibility of using, in the hair-dyeing product, MFC as cosmetic additive with multiple functions: suspending agent, stabiliser, conditioner, strengthener, and anti-drip agent.

### GENERAL CONCLUSIONS

On the basis of what has been analysed and provided in the examples described above, it is possible to draw the following conclusions: it is evident that MFC, in the case of shampoo formulations, leave-on hair-balm formulations, and hair-dyeing formulations (dyeing formulation and oxidant formulation), is able to achieve a good suspending and stabilising effect of the formulas cited above, also as compared to the synthetic suspending agents present on the market, as well as a conditioning effect, comparable, for example, to that of homocationic polymers, figuring amongst which is the polyquaternium class; this class of raw materials currently represents the class of conditioners most widely used in the cosmetic world for hair care; they presenting high performance, albeit having a negative impact on the environment on account of their low biodegradability and high toxicity in water.

In conclusion, through the preparation of formulations containing microfibrillated cellulose, it has been possible to demonstrate how acrylic polymers and polyquaternium can be replaced altogether or used in low percentages in combination with MFC to be able to satisfy the increasing demand of the market for highly sustainable cosmetic products and at the same time still boast high levels of performance both in terms of chemico-physical stability and in terms of final effect on the hair.

## Claims

1. Use of microfibrillated cellulose (MFC) as a conditioning and strengthening agent of hair, in a hair care cosmetic composition, wherein in the aforesaid hair care cosmetic composition:
a. the MFC is not chemically modified, and
b. the MFC is present in amounts from 0.1 wt% to 5 wt% by weight, preferably from 0.1 wt% to 3 wt% by weight, and wherein said hair care cosmetic composition does not contain acrylic (co)polymers and polymeric quaternary ammonium salts, and wherein the hair care composition is a shampoo, a leave on non-rinsing hair conditioner or a hair dye composition.

2. The use according to claim 1, wherein the hair dye composition is formed of a dye formulation to be mixed at the moment of use with an oxidising formulation, at least one of the two contains MFC and wherein the final mixture is an O/W emulsion.

3. The use according to claim 2, wherein the oxidising formulation, part of the dyeing hair composition, is formed of an aqueous phase containing at least one microfibrillated cellulose and obtained by monophase dispersion of the additives in the same.

4. The use according to any one of the preceding claims, wherein microfibrillated cellulose (MFC) fibrils have an average length from 100 nm to 50 µm, preferably from 1 µm to 10 µm, even more preferably from 3 µm to 10 µm, and an average diameter from 1 nm to 500 nm, preferably from 5 nm to 100 nm, even more preferably from 10 nm to 30 nm.

5. The use according to any one of the preceding claims, wherein the microfibrillated cellulose (MFC) has a water retention capacity of at least 50%, preferably at least 60%, even more preferably at least 70%, wherein the capacity to retain water is measured by diluting a sample of MFC up to 0.3 % weight in water and then subjecting the sample to centrifuging for 15' at 1000G, so that the supernatant is separated while the precipitate is weighted, wherein its capacity to retain water is obtained as (mV/mT) - 1, where mV is the weight of the wet precipitate and mT is the weight of the dried precipitate.

## Patentansprüche

1. Verwendung von mikrofibrillierter Cellulose (MFC) als Konditionierungs- und Stärkungsmittel für Haare in einer kosmetischen Haarpflegezusammensetzung, wobei in der besagten kosmetischen Haarpflegezusammensetzung:
a. die MFC nicht chemisch modifiziert ist, und
b. die MFC in Mengen von 0,1 Gew.-% bis 5 Gew.-%, vorzugsweise von 0,1 Gew.- % bis 3 Gew.-% vorhanden ist,
und wobei die besagte kosmetische Haarpflegezusammensetzung keine Acryl(co)polymere und polymeren quaternären Ammoniumsalze enthält, und wobei die Haarpflegezusammensetzung ein Shampoo, ein im Haar verbleibender (Leave-on) Haarkonditionierer oder ein Haarfärbemittel ist.

2. Verwendung nach Anspruch 1, wobei das Haarfärbemittel aus einer Färbeformulierung gebildet wird, die im Moment der Verwendung mit einer Oxidationsformulierung zu mischen ist, wobei mindestens eine der beiden MFC enthält und wobei die fertige Mischung eine O/W-Emulsion ist.

3. Verwendung nach Anspruch 2, wobei die Oxidationsformulierung, die Teil des Haarfärbemittels ist, aus einer wässrigen Phase gebildet wird, die mindestens mikrofibrillierte Cellulose enthält und durch einphasige Dispersion der Additive in derselben erhalten wird.

4. Verwendung nach einem der vorhergehenden Ansprüche, wobei die Fibrillen der mikrofibrillierten Cellulose (MFC) eine durchschnittliche Länge von 100 nm bis 50 µm, vorzugsweise von 1 µm bis 10 µm, noch bevorzugter von 3 µm bis 10 µm, und einen durchschnittlichen Durchmesser von 1 nm bis 500 nm, vorzugsweise von 5 nm bis 100 nm, noch bevorzugter von 10 nm bis 30 nm aufweisen.

5. Verwendung nach einem der vorhergehenden Ansprüche, wobei die mikrofibrillierte Cellulose (MFC) ein Wasserrückhaltevermögen von mindestens 50 %, vorzugsweise von mindestens 60 %, noch bevorzugter von mindestens 70 % aufweist, wobei das Vermögen, Wasser zurückzuhalten, gemessen wird durch Verdünnen einer MFC-Probe auf bis zu 0,3 Gew.-% in Wasser und anschließendes Zentrifugieren der Probe für 15 Minuten bei 1000G, so dass der Überstand abgetrennt wird, während das Präzipitat gewogen wird, wobei das Wasserrückhaltevermögen als (mV/mT) - 1 erhalten wird, wobei mV das Gewicht des feuchten Präzipitats ist und mT das Gewicht des getrockneten Präzipitats ist.

## Revendications

1. Utilisation de cellulose microfibrillée (MFC) en tant qu'agent de conditionnement et de renforcement des cheveux, dans une composition cosmétique pour le soin des cheveux, dans laquelle, dans ladite composition cosmétique pour le soin des cheveux :
a. la MFC n'est pas modifiée chimiquement, et
b. la MFC est présente dans des quantités allant de 0,1 % en poids à 5 % en poids, de préférence de 0,1 % en poids à 3 % en poids,
et dans laquelle ladite composition cosmétique pour le soin des cheveux ne contient pas de (co)polymères acryliques ni de sels d'ammonium quaternaire polymères, et dans laquelle la composition pour le soin des cheveux est un shampooing, un après-shampooing sans rinçage ou une composition de teinture capillaire.

2. Utilisation selon la revendication 1, dans laquelle la composition de teinture capillaire est formée d'une formulation de teinture à mélanger au moment de l'utilisation avec une formulation oxydante, au moins l'une des deux contenant de la MFC et dans laquelle le mélange final est une émulsion H/E.

3. Utilisation selon la revendication 2, dans laquelle la formulation oxydante, partie de la composition de teinture capillaire, est formée d'une phase aqueuse contenant au moins de la cellulose microfibrillée et obtenue par dispersion monophasique des additifs dans celle-ci.

4. Utilisation selon l'une quelconque des revendications précédentes, dans laquelle les fibrilles de cellulose microfibrillée (MFC) présentent une longueur moyenne allant de 100 nm à 50 µm, de préférence de 1 µm à 10 µm, plus préférablement de 3 µm à 10 µm, et un diamètre moyen allant de 1 nm à 500 nm, de préférence de 5 nm à 100 nm, plus préférablement de 10 nm à 30 nm.

5. Utilisation selon l'une quelconque des revendications précédentes, dans laquelle la cellulose microfibrillée (MFC) présente une capacité de rétention d'eau d'au moins 50 %, de préférence d'au moins 60 %, de manière plus préférable d'au moins 70 %, dans laquelle la capacité à retenir l'eau est mesurée en diluant un échantillon de MFC jusqu'à 0,3 % en poids dans de l'eau et en soumettant ensuite l'échantillon à une centrifugation pendant 15 minutes à 1000G, de sorte que le surnageant est séparé tandis que le précipité est pesé, dans laquelle sa capacité à retenir l'eau est obtenue sous la forme (mV/mT) - 1, où mV est le poids du précipité humide et mT est le poids du précipité séché.
